# EUROPEAN PATENT SPECIFICATION

(11) **EP 1 659 958 B1**
(45) Date of publication and mention of the grant of the patent: **25.07.2012**
(21) Application number: 04781135.1
(22) Date of filing: 13.08.2004
(51) Int. Cl.: A61B 17/17

(54) **QUICK-RELEASE DRILL GUIDE ASSEMBLY FOR BONE PLATE**
BOHRFÜHRUNGSANORDNUNG MIT SCHNELLER AUSLÖSUNG FÜR KNOCHENPLATTE
ENSEMBLE GUIDE DE MECHE A LIBERATION RAPIDE POUR PLAQUE VISSEE

(30) Priority: 13.08.2003 US 639515
(43) Date of publication of application: 31.05.2006
(73) Proprietor: Synthes GmbH, 4436 Oberdorf (CH)
(72) Inventor: BINDER, Lawrence, J., Jr., Doylestown, PA 18901 (US); RYAN, Christopher, J., West Chester, PA 19380 (US); STIHL, Pascal, 2540 Grenchen (CH)
(74) Representative: Klunker . Schmitt-Nilson . Hirsch
(86) International application number: PCT/US2004/026399
(87) International publication number: WO 2005/016128

(56) References cited:
- WO-A1-01/82804
- US-A- 5 154 720
- US-A- 5 851 207
- US-B1- 6 342 057

## Description

### FIELD OF THE INVENTION

The present invention relates to a surgical drill-guide assembly that can be releasably attached to a part of a bone-fixation system, for example, a bone plate. The surgical drill-guide assembly of the present invention is used for example, to guide a drill-bit, screw, bone fastener, or other instrument or fastener into bone or other tissue.

### BACKGROUND OF THE INVENTION

The use of surgical fixation plates for a variety of orthopedic applications Is widely accepted. The plates are used by surgeons or users to stabilize, mend, or align a patient's bone as well as alter compression of patient's bones. Plates are typically fastened to the bones with a plurality of fasteners such as screws that are installed through holes in the plate. Proper orientation and alignment of fasteners and secure surgical fixation of the plates can mitigate some of the potential future complications after implantation.

Bone plates used, for example, in spinal applications must be installed with special care, as the plates may be used for long-term, intervertebral fixation, bone-fragment fixation, and/or anterior decompression in the cervical region of the spine. The margin for error in spinal surgery is quite small, particularly because of the sensitivity of the spinal cord and the risk inherent with invasive procedures around the spinal cord. In particular, the dimensions of vertebral bone available for setting fasteners are fairly limiting.

Each fixation screw should property align with its associated plate hole so that each screw is seated correctly with the plate and enters the bone at an appropriate angle. Any misalignment of the screw within the plate hole risks tissue damage and spinal cord injury. In addition, improperly seated screws may result in an unstable or insecure connection of the plate to the bony material, thus potentially defeating the usefulness of the plate. Locking plates, in particular, demand precise fastener alignment.
WO 01/82804 A1 discloses a drill-guide assembly which is releasably lockable to the fastener holes of a bone plate. The drill-guide assembly comprises two alignment barrels and respective radially expandable bushings to slidably receive the barrels. The bushings are expanded by advancing the barrels through the bushings. The barrels are advanced by means of an actuation bar which is connected to the barrels and operated by squeezing the handle of the drill-guide assembly. The advanced position and thereby locking of the bushings in the fastener holes may be maintained by pushing a latch to hold the actuation bar in position.

### SUMMARY OF THE INVENTION

The present invention relates to a drill-guide assembly, which in one embodiment comprises an alignment drill-barrel, a bushing, a dual-arm support, a ratchet-gear mechanism, a handle member, and a release knob.

The alignment drill-barrel has a proximal end and a forward-end also called the distal end. The proximal end of the alignment drill-barrel preferably has two ridges, and the distal end is generally tapered. The alignment drill-barrel is configured to receive and guide a drill-bit, bone tap, screw, bone fastener or other instrument into bone or other tissue. The alignment drill-barrel preferably allows for the passage of fixation pins or bone screws, drills, taps, or awls through it in a predetermined trajectory.

The bushing preferably has a radially expandable forward-end and a proximal end, wherein the forward-end is configured to engage a fastener hole in a bone-plate. The radially expandable forward end of the bushing preferably has a plurality of finger portions. The radially expandable forward end also preferably has a shoulder, neck, and an outwardly projecting rim disposed forward of the neck. The bushing is configured to slidably receive the alignment drill-barrel. Sliding the alignment drill-barrel toward the forward end of the bushing preferably expands the forward end of the bushing to secure the drill-guide assembly in a bone-plate.

The dual-arm support in one embodiment is generally "L-shaped" with the two ends of the "L" forming an obtuse angle. The dual-arm support preferably has a space provided in its center region. In one embodiment, the end portion, which is generally horizontally disposed, comprises a pivot-hole for inserting a pivot screw. At one end, the dual-arm support is immovably or fixedly connected to the proximal end of the bushing, while at its other end, the dual-arm support is immovably connected to the front end of the handle member.

The handle member in an exemplary embodiment has a front end and a back end. It is generally oval shaped with broad grooves on top to provide better grip for the surgeon or user using the drill-guide assembly. The handle may be hollow or solid depending upon design choice.

The ratchet-gear mechanism in one embodiment is generally "Y-shaped" and is housed within the space of the dual-arm support. At one end, the first leg of the ratchet-gear mechanism is pivotably connected to the dual-arm support at a pivot-point. That end of the first leg further extends beyond the pivot point forming a C-shaped vice-grip. The C-shaped vice-grip attaches to the alignment drill-barrel. The C-shaped vice-grip grasps the alignment drill-barrel in between the two ridges at the proximal end. In a preferred embodiment, the plane of the C-shaped vice-grip is generally perpendicular to the axial direction of the alignment drill-barrel, and the bushing. The second leg of the Y-shaped ratchet-gear mechanism comprises pawls on the outer side which permit incremental swiveling of the ratchet-gear mechanism in a plane perpendicular to the plane of C-shaped vice-grip. The tail-end of the Y-shaped ratchet-gear mechanism acts as a trigger and generally moves in a rotational motion relative to the pivot point in a direction toward or away from the handle member. Movement of the ratchet-gear mechanism, and particularly the C-shaped vice grip, slides the alignment drill-barrel relative to the bushing.

The release knob in an exemplary embodiment has a curved longitudinal member with a base. The base has serrations on one side of its circumferential border and a hole on the other side. The release knob is pivoted through the hole in the base about a dowel pin that is attached to the dual-arm support.

When the tail of the Y-shaped ratchet-gear mechanism is pressed by a finger of a user in a rotary motion in a direction toward the handle member, the distal end of the alignment drill-barrel is urged into the bushing which in turn, expands the forward-end of the bushing, thus locking the bushing within a hole or recess of the bone-plate. The bushing is configured and dimensioned to expand within a bone-plate hole or recess such that it is releasably locked to the bone-plate.

When the Y-shaped ratchet-gear mechanism engages the release knob, the pawls on the outer surface of the second leg of the Y-shaped ratchet-gear mechanism engage the serrations on the release knob to lock the drill-guide to the bone-plate. The alignment drill-barrel preferably self-aligns with the axis of the fastener hole in the plate.

When the release knob is further pressed, the pawls are disengaged from the serrations, and the Y-shaped ratchet-gear mechanism returns to an unactuated position, preferably by action of a biasing member such as a spring. The Y-shaped ratchet-gear mechanism, in turn, through its C-shaped vice-grip moves the alignment drill-barrel in a longitudinal direction along its axis, away from the fingers. As a result, the bushing assumes a retracted position thereby disengaging the hole or recess.

Another embodiment of a drill-guide assembly is described, comprising an alignment barrel having a proximal end and a distal end; a bushing configured to slidably receive the alignment barrel, the bushing having a radially expandable forward-end and a proximal end, the forward-end configured to be insertable within a hole or recess in a bone plate; a release knob having serrations; and a movable ratchet gear mechanism having a first leg, a second leg and a tail, the first leg of the ratchet-gear mechanism connected to the alignment barrel, the second leg of the ratchet-gear mechanism having pawls configured and adapted to engage the serrations to hold the alignment barrel in position, the tail of the ratchet gear mechanism operable by a user to selectively move the ratchet-gear mechanism, wherein, movement of the ratchet-gear mechanism slides the alignment barrel relative to the bushing to radially expand the forward end to releasably lock the bushing to the plate, and a first drill guide coupled to the bushing, wherein the first drill guide is configured to receive and guide a drill-bit.

The first drill guide may be coupled to the bushing by a first connecting element. The first connecting element may have at least two bores for respectively receiving at least a portion a bushing therethrough and at least a portion of a drill guide therethrough. The first drill guide may also be further coupled to the bushing by a second connecting element, and the second connecting element may have at least two bores for respectively receiving at least a portion of a bushing therethrough and at least a portion of a drill guide therethrough.

At least two bores of the first connecting element may be separated by a first distance, and the at least two bores of the second connecting element may be separated by a second distance, wherein the first distance may be greater than the second distance, and wherein the second connecting element may be closer to the distal end of the forward end of the bushing than the first connecting element. Alternatively, the first connecting element may be closer to the distal end of the forward end of the bushing than the second connecting element.

The first connecting element may further comprise a fin bore configured to receive at least a portion of fin therethrough, wherein at least a portion of the fin in configured to engage at least a portion of a hole or recess when the bushing engages a bone-plate.

The drill-guide assembly may further comprise a second guide coupled to the bushing, wherein the second drill guide is configured to receive and guide a drill-bit. The second drill guide may be coupled to the first drill guide. The first and second drill guide may be coupled to the bushing by a first connecting element. The first connecting element may have at least three bores for respectively receiving at least a portion of the first drill guide therethrough, at least a portion of the second drill guide therethrough, and a least a portion of the bushing therethrough.

The first connecting element may further include a fin bore configured to receive at least a portion of fin therethrough, wherein at least a portion of the fin in configured to engage at least a portion of a hole or recess when the bushing engages a bone-plate.

The first drill guide and second drill guide may further be coupled to the bushing by a second connecting element.

The second connecting element may also have at least three bores for respectively receiving at least a portion of the first drill guide therethrough, at least a portion of the second drill guide therethrough, and a least a portion of the bushing therethrough.

The bores of the first connecting element receiving first and second drill guides are separated by a first distance, and the bores of the second connecting element receiving first and second drill guides are separated by a second distance, wherein the first distance is greater than the second distance, and wherein the second connecting element may be closer to the distal end of the forward end of the bushing than the first connecting element. Alternatively, the first connecting element may be closer to the distal end of the forward end of the bushing than the second connecting element.

The first drill guide may have a longitudinal axis, and when the bushing is locked to a bone-plate, the longitudinal axis of the first drill guide may generally be aligned with a first bone-fastener hole of the bone-plate. The drill-guide assembly may further comprise a second drill guide configured to receive and guide a drill-bit and coupled to the bushing, the second drill guide having a longitudinal axis, and when the bushing is locked to a bone-plate, the longitudinal axis of the second drill guide may be generally aligned with a second bone-fastener hole of the bone-plate. The recess of the bone-plate includes at least one shaped area and a slot.

An alternative method for drilling holes in bone is also described, comprising the steps of: (a) providing a drill-guide assembly, comprising an alignment barrel having a proximal end and a distal end; a bushing configured to slidably receive the alignment barrel, the bushing having a radially expandable forward-end and a proximal end, the forward-end configured to be insertable within a hole or recess in a bone plate; a release knob having serrations; and a movable ratchet gear mechanism having a first leg, a second leg and a tail, the first leg of the ratchet-gear mechanism connected to the alignment barrel, the second leg of the ratchet-gear mechanism having pawls configured and adapted to engage the serrations to hold the alignment barrel in position, the tail of the ratchet gear mechanism operable by a user to selectively move the ratchet-gear mechanism, wherein, movement of the ratchet-gear mechanism slides the alignment barrel relative to the bushing to radially expand the forward end to releasably lock the bushing to the plate, and at least a first guide coupled to the bushing, wherein the first drill guide is configured to receive and guide a drill-bit; (b) inserting the bushing into a recess of a bone plate; (c) aligning at least the first drill guide with a first bone fastener hole in the bone-plate; (d) expanding the bushing in the recess; (e) locking the bushing to the plate; (f) inserting a drill-bit into the first drill guide; and (g) drilling a first hole.

At least a portion of the forward end of the bushing may be configured to fit in at least a portion of the recess. The bushing may be locked to the plate by locking the alignment barrel and bushing in fixed relation to each other. The drill guide assembly may further comprise a second drill guide coupled to the bushing, wherein the second drill guide is configured to receive and guide a drill-bit.

The method may further comprise the steps of inserting a drill-bit guide into the second drill guide, and drilling a second hole.

A kit for use with drilling bones is also described, comprising: (a) a drill-guide assembly, comprising an alignment barrel having a proximal end and a distal end; a bushing configured to slidably receive the alignment barrel, the bushing having a radially expandable forward-end and a proximal end, the forward-end configured to be insertable within a hole or recess in a bone plate; a release knob having serrations; and a movable ratchet gear mechanism having a first leg, a second leg and a tail, the first leg of the ratchet-gear mechanism connected to the alignment barrel, the second leg of the ratchet-gear mechanism having pawls configured and adapted to engage the serrations to hold the alignment barrel in position, the tail of the ratchet gear mechanism operable by a user to selectively move the ratchet-gear mechanism, wherein, movement of the ratchet-gear mechanism slides the alignment barrel relative to the bushing to radially expand the forward end to releasably lock the bushing to the plate; (b) at least first and second drill guides able to be coupled to the bushing; and (c) at least first and second connecting elements for coupling at least one drill guide to the bushing.

At least the first and second drill guides may have different lengths, and at least the first and second drill guides may have different diameters. At least the first and second connecting elements may each have a bore for receiving at least one drill guide therethrough and a bore for receiving a bushing therethrough, wherein the bores of the first connecting element have a first arrangement, and the bores of the second connecting element have a second arrangement, and wherein the first arrangement may be substantially different than the second arrangement.

### BRIEF DESCRIPTION OF THE DRAWINGS

Preferred features of the present invention are disclosed in the accompanying drawings, wherein similar reference characters denote similar elements throughout the several views. While the presentation is desired and its features presented according to certain illustrated embodiments it is to be understood that the invention is not so limited to the particular embodiments shown and described, wherein:

FIG. 1 is a perspective view of a first embodiment of a drill-guide assembly;

FIG. 2 is a cross-sectional view of an embodiment of an alignment drill-barrel that may be used with the assembly of FIG. 1;

FIG. 3 is a partial cross-sectional view of another embodiment of an alignment drill-barrel that may be used with the assembly of FIG. 1;

FIG. 4 is a cross-sectional view of an embodiment of the bushing;

FIG. 5 is a side view of the dual-arm support attached to the bushing and handle member;

FIG. 6 is a perspective view of the Y-shaped ratchet-gear mechanism;

FIG. 6A is a side view of the Y-shaped ratchet-gear mechanism;

FIG. 6B is a perspective view of the drill-guide assembly showing the ratchet-gear mechanism connected to the dual-arm support;

FIG. 7 is a side view of the release knob;

FIG. 7A is a perspective view of the ratchet-gear mechanism engaging the release knob;

FIG. 8 is a side view of the handle member of the drill-guide assembly;

FIG. 9 is a side view of the bushing with fingers in retracted position;

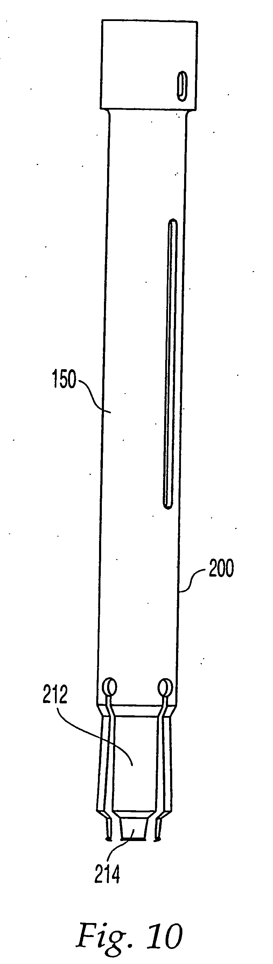

FIG. 10 is a side view of the bushing with fingers in expanded position; and

FIG. 11 is a perspective view of the drill-guide assembly of FIG. 1 engaged to a bone-plate.

FIG. 12A is a perspective view of another embodiment of a drill-guide assembly with drill guides;

FIG. 12B is another perspective view of the assembly of FIG. 12A;

FIG. 12C is a partial top view of an exemplary bone plate that can be used with the assemblies of FIGS. 1 and 12A;

FIG. 13A is a top view of a proximal connecting element for use with the assembly of FIG. 12A; and

FIG. 13B is a top view of a distal connecting element for use with the assembly of FIG. 12A.

### DETAILED DESCRIPTION OF THE INVENTION

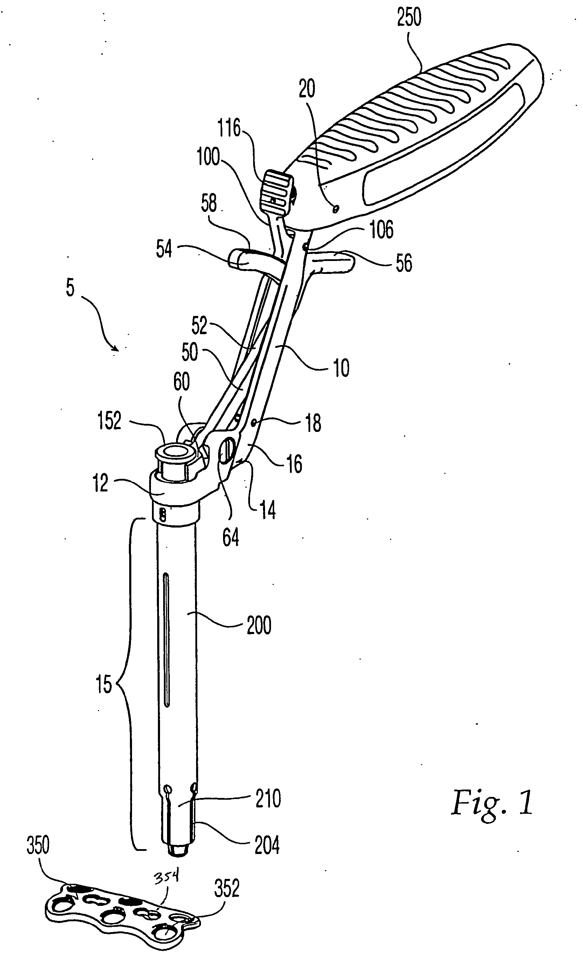

Referring to FIG. 1, there is shown an exemplary surgical drill-guide assembly 5, which is adapted for use with a cervical spine-locking bone plate having a plurality of fastener holes. While the surgical drill-guide assembly is described in conjunction with a cervical locking plate it will be appreciated that the reference to a cervical locking plate is only exemplary, and that the surgical drill-guide assembly can be used with a variety of bone plates, including a locking and a nonlocking bone-plate as well as for example, bone plates for long bones, maxillofacial applications, etc.

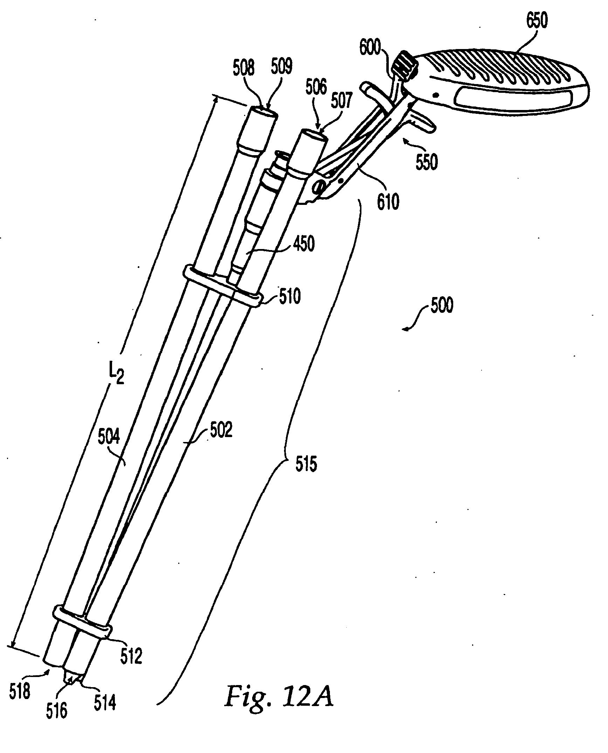

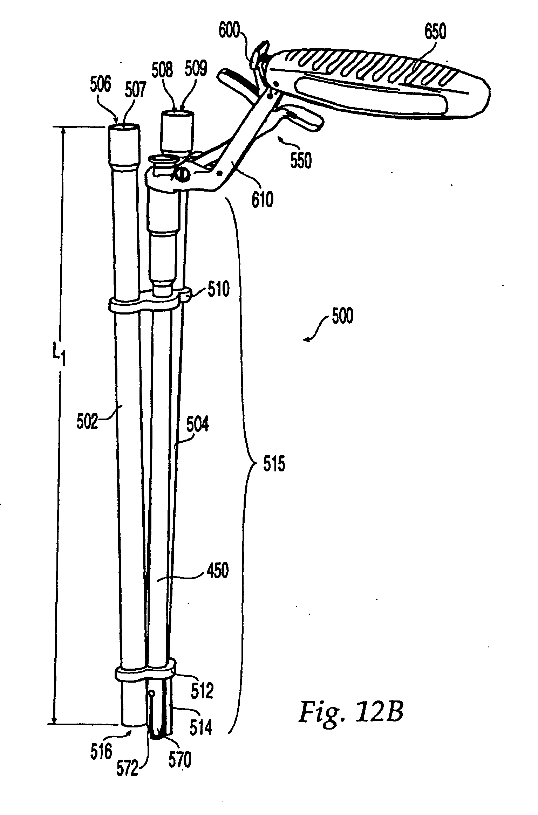

This embodiment of a drill-guide assembly 5 can be secured or locked into a fastener hole in a bone plate. A related embodiment of a drill-guide assembly 500 that can be secured or locked into a drill recess 354 is shown *infra* in FIGS. 12A-13B. Locking or securing may facilitate precision in the surgical procedure, for example, drilling or fastening screws or other similar fasteners. Moreover, the drill-guide can be quickly detached and released from the bone-plate improving the speed of surgical procedures involving drilling or similar procedures.

Drill-guide assembly 5 may include an alignment assembly 15, a release knob 100, a handle member 250, a ratchet-gear mechanism 50, and a dual-arm support 10.

The alignment assembly 15 may comprise an alignment drill-barrel 150 and a bushing 200. A surgeon or a user can releasably attach the alignment assembly 15 in the fastener hole 352 of a bone-plate 350. Other attachment options are discussed *infra,* particularly in relation to FIGS. 12A-13B. A drill-bit or other such instrument can be inserted into and through the alignment assembly 15.

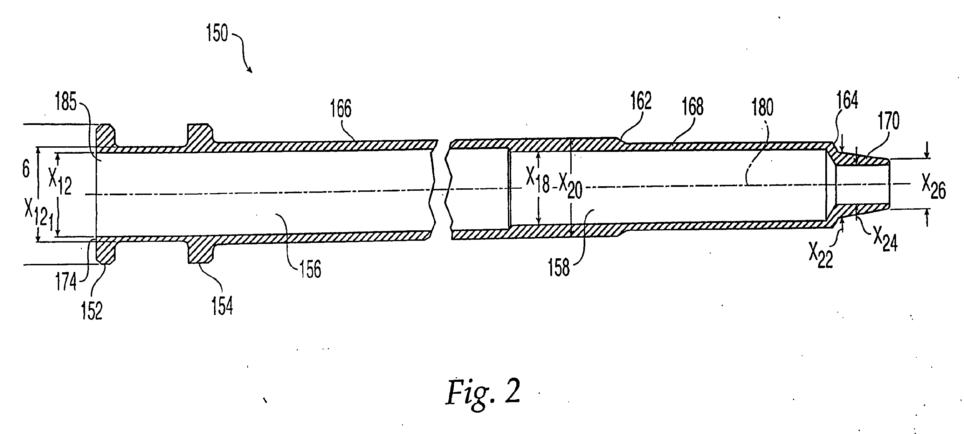

Referring to FIG. 2, an embodiment of the alignment drill-barrel 150 is shown. The alignment drill-barrel 150 may have a through bore 185 from its proximal end 174 to its distal end 172. A drill-bit or other instrument may be inserted through the bore 185. In the embodiment of FIG. 2, the drill-barrel comprises a first hollow cylindrical section 156 with an annular diameter of x₁₂, a second hollow cylindrical section 158 with an inside annular diameter of x₁₈, and a third hollow cylindrical section 160 with an inside annular diameter of x₂₄, wherein x₂₄ is smaller than x₁₈, and x₁₈ is smaller than x₁₂. The outside surface of the alignment drill-barrel 150 comprises a shoulder 162 and a shoulder 164 wherein the outside diameter of the first section 166 is x₁₄ which is greater than the outside diameter x₂₀ of the second section 168. x₁₄ has an exemplary diameter of 3 mm to 10 mm, preferably about 8 mm. The third section 170 is a conical section that tapers from an outside diameter x₂₂ at shoulder 164 to a diameter x₂₆ at the distal end 172. The proximal end 174 of the alignment drill-barrel 150 preferably has first circular ridge 152 and second circular ridge 154. The first and the second circular ridges 152 and 154 respectively, have an outside diameter x₁₆.

In this embodiment, the first circular ridge 152 is flush with the proximal end 174 of the alignment drill-barrel 150. The conical section 170 tapers from an outside diameter x₂₂ at the transition 164 to an outside diameter x₂₆ at end 172. Preferably, inner diameter x₂₄ is constant along the length of conical section 170 of alignment drill-barrel 150 as defined along center line 180.

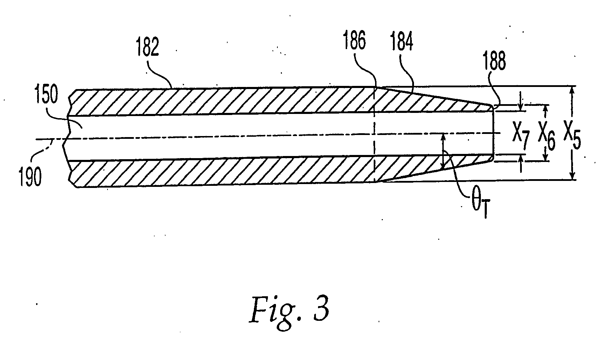

Referring to FIG. 3, an alignment drill-barrel 150 according to another embodiment is shown. In FIG. 3, alignment drill-barrel 150 is hollow with a cylindrical section 182 and a tapered, conical section 184 to facilitate movement of alignment drill-barrel 150 within bushing 200. Cylindrical section 182 has outside diameter x₅, while conical section 184 tapers from an outside diameter x₅ at the transition 186 to an outside diameter x₆ at the distal end 188. Preferably, inner diameter x₇ may be constant along the length of alignment drill-barrel 150 as defined along center line 190.

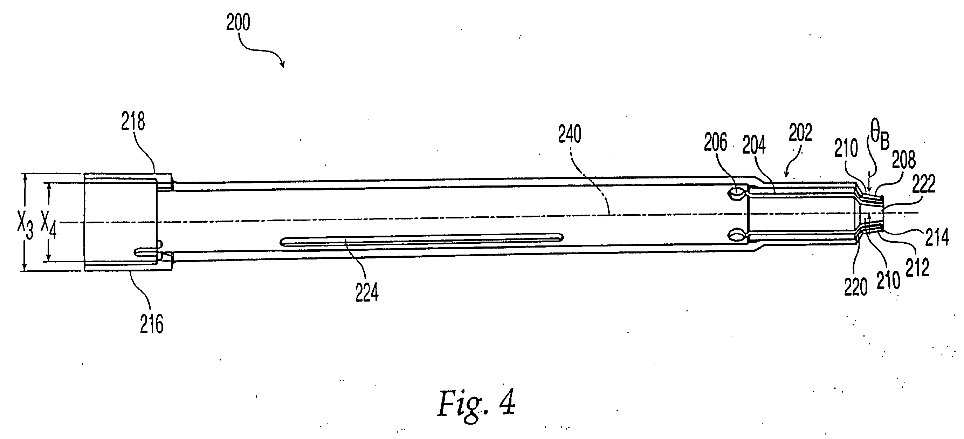

Referring to FIG. 4, a bushing is shown. Bushing 200 may coaxially receive alignment drill-barrel 150 about a central line 240. Bushing 200 may be substantially symmetrical about line 240. The forward end 222 of bushing 200 may preferably be comprised of longitudinally extending fingers 210. Individual fingers 210 may be separated by slits 204 extending longitudinally between adjacent fingers 210. Slits 204 as shown, for example, in FIG. 4, may include a circular portion 206 that serves to minimize stress concentration when fingers 210 are flexed. Fingers 210 may be resiliently biased inwardly and naturally assume an inward disposition when in a relaxed state. At a front portion of the expandable forward end 202 of bushing 200, the fingers 210 may form a radially expandable circumferential neck 208. At the back end of and adjacent to neck 208 may preferably be a shoulder 212.

Neck 208 may span a length that is slightly longer than the thickness of the fastener hole wall from the bone-side surface to the top surface of a bone-plate. Thus, neck 208 can be inserted into the bone-plate fastener hole 352 and the fingers 210 expanded to secure the bushing 200 to the plate. More particularly, movement of alignment drill-barrel 150 within bushing 200 may expand fingers 210 to secure the bushing 200 to the bone plate. In this manner, the drill-guide assembly can be secured to the plate, restricting relative movement. In a preferred embodiment, fingers 210 forming a radially expandable rim 214 may be provided at the front end of and adjacent to neck 208.

In another embodiment, the distal end 222 of the bushing 200 may not contain the rim 214, the neck 208 or the shoulder 212, but instead has a tapered end with the inner and the outer diameter of the tapered end decreasing from point 220 shown in FIG. 4. In such an embodiment, the taper is such that it may fit freely through a fastener hole in a bone plate.

In alternate embodiments, no rim may be used. The several portions of bushing 200, i.e., the neck 208, the shoulder 212, and the rim 214, may preferably be a single piece of material of unitary construction.

In other alternate embodiments, fingers 210 need not include a shoulder, neck, and/or a rim. Instead, for example, a small pin may be used to secure the bushing to the plate. In an alternatively preferred embodiment, the inward bias of fingers 210 is selected to produce the desired friction with the bone-plate 350 so that the fingers 210 fit snugly within the bone-plate fastener hole 352 (or drill recess 354, as discussed *infra* in relation to FIGS. 12A-13B), preferably allowing operation of handle member 250 with only one hand. Alternative resiliency for fingers 210 may be varied to suit the purpose of the design.

In a preferred embodiment bushing 200 has one or more longitudinal slots on its side 224 in axial direction 240 just above the circular portion 206. These slots provide better cleaning during autoclave or other disinfection and/or cleaning procedures.

Referring to FIG. 4, bushing 200 has a circumferential ridge 218 with an outer diameter x₃, and a region 216 has an outer diameter x₄. x₄ has an exemplary dimension of 4 mm to 20 mm, preferably about 8 mm.

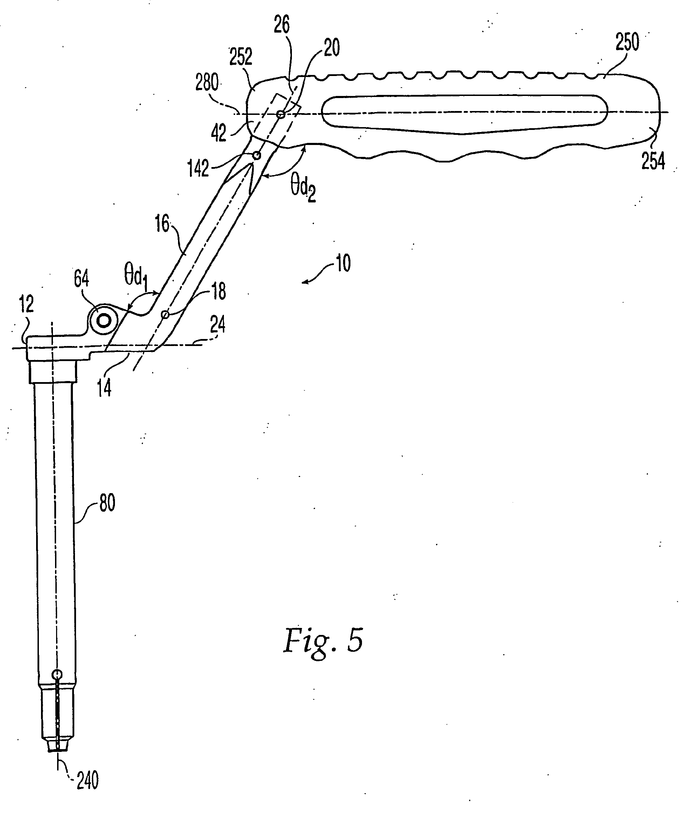

As shown in FIG. 5, in one embodiment, dual-arm support 10 connects the handle member 250 to the alignment assembly 15. More specifically, in the exemplary embodiments of FIGS. 1 and 2, the dual-arm support 10 is fixedly connected at its end to the proximal end 174 of the alignment assembly 15. Dual-arm support 10 preferably is generally "L-shaped" with first part 14 connected to bushing 200. More specifically, end 12 of dual-arm support 10 is attached to ridge 218 at the proximal end 242 of the bushing 200.

The dual-arm support 10 is preferably fixed with the bushing 200 by welding. In an alternative embodiment, friction fitting, press fitting, and such can be used. Outer diameter x₃ of ridge 218 is about the same size as inner diameter x₁ of the clamp 12 of the dual-arm support 10. Bushing 200 may also be fixed to dual-arm support 10 by releasable fastener means. First part 14 is generally perpendicular to the axial direction of the alignment assembly 15 or the bushing 200. The second part 16 of the dual-arm support 10 preferably forms an obtuse angle θ_{d1} with the first part 14 of the dual-arm support 10. θ_{d1} may range from about 90° to about 180º, and more preferably from about 105º to about 135º. Dual-arm support 10 and handle member 250 are fixedly connected by a dowel pin 20 at the front end of the handle member 250, so that they are immovable with respect to each other. In the preferred embodiment, handle member 250 is located remotely from the drilling site, thereby increasing visibility near the locking bone plate 350.

As shown in FIG. 5, the second part 16 of the dual-arm support 10 may be attached to the first part 14 by a dowel pin 18, or the dual-arm support 10 may be an integral, monolithic construction. The second part 16 of the dual-arm support 10 also forms an obtuse angle θ_{d2} with the handle member 250. θ_{d2} may range from about 90° to about 180º, and more preferably from about 105º to about 135º. The handle member 250 and the dual-arm support 10 generally form an "S" shape or a zigzag shape, and in a preferred embodiment, the longitudinal axis 24 of the first part 14 and the longitudinal axis 26 of the second part 16 lie in the same plane. The longitudinal axis 280 of the handle member 250 also preferably lies in the same plane as the longitudinal axis 24 of the first part 14 and the longitudinal axis 26 of the second part 16 of the dual-arm support 10. Preferably the longitudinal axis 24 of the first-part 14 of the dual-arm support 10 is generally parallel with the longitudinal axis 280 of the handle member 250.

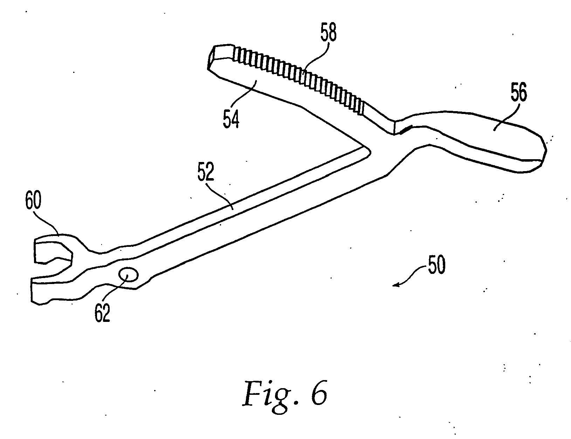

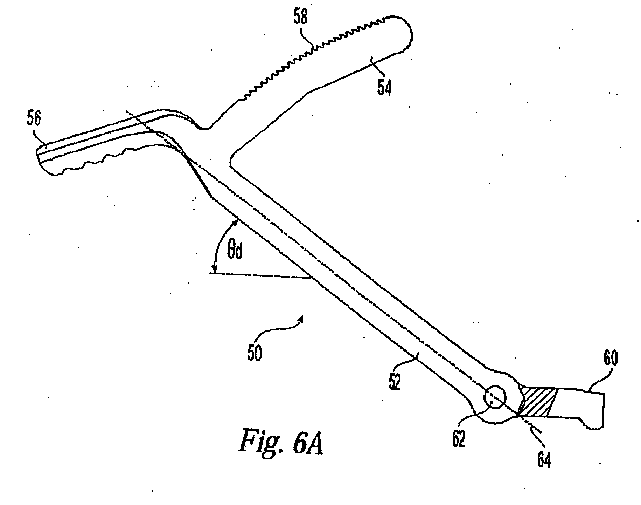

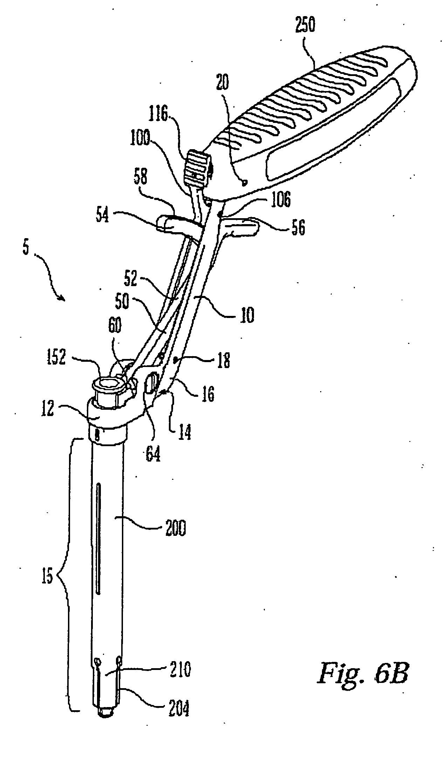

Referring to FIG. 6, there is shown an exemplary embodiment of the ratchet-gear mechanism 50. The ratchet-gear mechanism 50 allows the user to manipulate the locking and release of the drill-guide assembly 5 with the bone-plate 350 by engagement and disengagement, respectively, of the pawls 58 with the serrations 102. The ratchet-gear mechanism 50, in a preferred embodiment is generally "Y-shaped" with a first leg 52, a second leg 54, and a tail 56.

The first leg 52 of the ratchet-gear mechanism comprises a generally C-shaped vice-grip 60 at its end, and a pivot hole 62 for insertion of a pivot screw 64. The C-shaped vice-grip 60 grips the alignment drill-barrel 150 in between the first ridge 152 and second ridge 154 (see also FIG. 2) located at the end 174 of the drill-barrel 150. As shown in FIG. 6A, in a preferred embodiment, the plane of the C-shaped vice-grip 60 that forms an anterior portion of the first leg 52 of the Y-shaped ratchet-gear mechanism 50 makes an acute angle θ_{d} with the longitudinal axis 64 of the first leg 52 of the Y-shaped ratchet-gear mechanism 50. At the point of inflexion between the longitudinal first leg 52 and the C-shaped vice grip 60, pivot screw 64 and hole 62 are located. This pivot mechanism 62 helps the movement of the alignment drill-barrel 150. In a preferred embodiment, the acute angle is from about 25° to about 45°. In a further preferred embodiment the acute angle θ_{d} is such that when the ratchet-gear mechanism 50 is completely disengaged from the serrations 102 of the release knob 100, the alignment drill-barrel 150 can be removed from the bushing 200 in a longitudinal direction away from the fingers 210 by moving the ratchet-gear mechanism 50 in a direction away from the handle member 250, about the pivot screw 64. θ_{d} may be 0º to 90º, with an exemplary dimension of 60º.

The second leg 54 of the Y-shaped ratchet-gear mechanism 50 comprises horizontal pawls 58 which engage serrations 102 at the end of the release knob 100. The tail 56 of the Y-shaped ratchet-gear mechanism 50 acts as a trigger for a user to apply a force to actuate movement of the alignment drill-barrel 150.

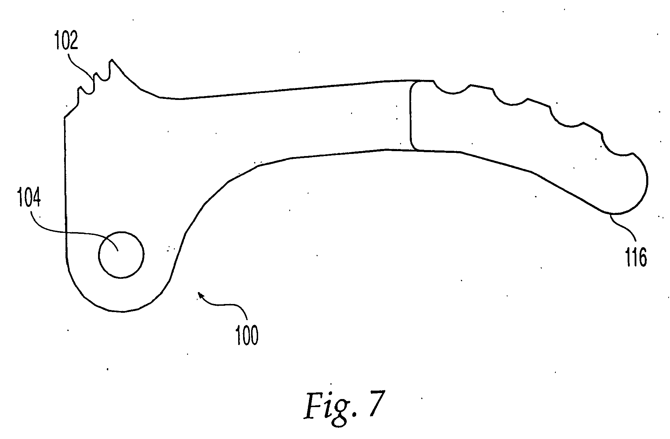

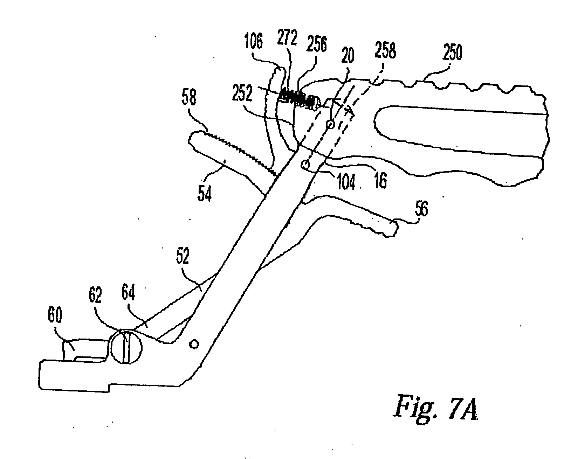

Referring to FIG. 7, the release knob 100 is pivoted about a dowel pin 106 which is inserted through the dowel pin hole 104 in the release knob 100, and the release knob hole 142 in the second part 16 of the dual-arm support 10. With pivotal support from the dowel pin 106, the serrations 102 on the surface of the release knob 100 can engage with the pawls 58 on the second leg 54 of the Y-shaped ratchet-gear mechanism, when the tail 56 (trigger) of the Y-shaped ratchet-mechanism is pressed or moved in a direction toward the handle member 250. In a preferred embodiment, the release knob 100 has a rubber sleeve 106 or a sleeve made from a material which provides a firm traction when the surgeon or the user presses the release knob 100. Alternatively, or additionally the surface of the release knob may have surface texturing to increase the traction when a surgeon or a user manipulates the release knob 100.

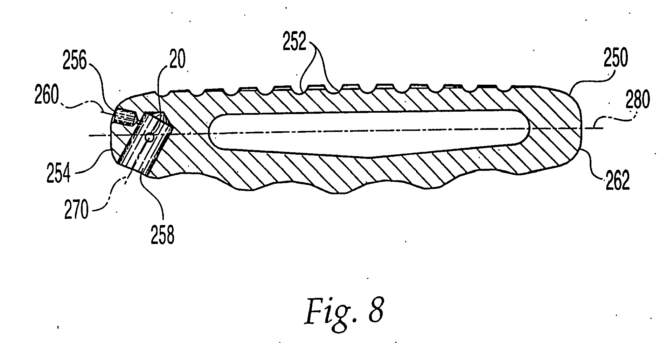

Referring to FIG. 8, handle member 250 is shown. Handle member 250 is generally oval shaped with broad grooves 252 on top to provide better grip to the surgeon or user when using the drill-guide assembly 5. At the front end 254 of the handle member 250, there are two cavities, the first cavity 256 and the second cavity 258. The first cavity 256 has an axis along line 260 and the second cavity 258 has an axis along line 270. The first cavity 256 houses compression spring 272 and the second cavity 258 houses the dual-arm support 10, or more specifically the second part 16 of the dual-arm support 10. The second part 16 of the dual-arm support 10 is fixed to the handle member 250 by a dowel pin 20. The dowel pin 20, in a preferred embodiment, is generally perpendicular to the axis 280 of the handle member 250. Exemplary dimensions of the handle are 100 to 150 mm long with a width at the widest point of 15 mm to 40 mm.

When a surgeon or a user presses the trigger 56, toward handle member 250, the ratchet-gear mechanism 50 swivels. Due to the movement of the Y-shaped ratchet-gear mechanism 50 in the direction of the handle member 250, the alignment drill-barrel 150 moves the bushing 200 in the downward direction toward the bone-plate 350. Due to the conical shape 170 of the alignment drill-barrel 150 (FIG. 2), the fingers 210 on the bushing 200 expand in an outward direction as the front end 172 of alignment drill-barrel 150 approaches the front edge 214 of bushing 200. When the outward diameter of the fingers 210 matches that of the fastener hole 352, the drill-guide assembly 5 locks to the bone-plate 350. A surgical drill-bit 400 or any other appropriate bit, screw, tap, awl, or such device, can be inserted through the alignment drill-barrel 150.

Alignment drill-barrel 150 may be configured and dimensioned to be slidably received within bushing 200. The alignment drill-barrel 150 and bushing 200 may cooperate to permit drill-guide assembly 5 to lock to a bone plate 350. The conical section 184 of the alignment drill-barrel 150 may cooperate with fingers 210 of bushing 200 to expand fingers 210 when the alignment drill-barrel 150 is moved into a locked position. The conical section 184 of alignment drill-barrel 150 may push outwardly against the inner surface of the bushing 200 as alignment drill-barrel 150 is moved forward to expand the forward end 214 of the bushing 200. In this embodiment, the conical section mates with and pushes against the inner surface of the bushing 200 forward of circular portion 206 of slits 204 in fingers 210, to push the fingers 210 radially outward (see FIG. 4).

Alignment drill-barrel 150 may be aligned within bushing 200, such that center line 240 or 190 may be collinear with line 180. When bushing 200 is placed in a fastener hole of a bone plate, and ratchet-gear mechanism 50 is actuated such that the almost fully actuated position is reached (i.e. when trigger 56 is substantially parallel to handle member 250), end 172 of alignment drill-barrel 150 may be substantially coplanar with rim 214 of bushing 200. It should be noted that alignment drill-barrel 150 may be coaxially received in bushing 200 which may also be the path of surgical drill-bit 400 inserted in cannula 182 of the alignment drill-barrel 150.

Generally, a surgeon or user should continue to depress the trigger 56 and handle member 250 toward each other to maintain an actuated position of Y-shaped ratchet-gear mechanism. Depending on the size of the fastener hole 352 (or drill recess 354, see FIGS. 12A-13B) and the firmness of the locking desired, the pawls 58 located on the second leg 54 of the Y-shaped ratchet-gear mechanism 50 may engage with the serrations 102 on the release knob 100 holding the ratchet-gear mechanism 50 in place. The release knob 100 preferably may be held firm in its position by the compression force of the spring mechanism 272, which may be located at the front end 254 inside the cavity 256 of the handle member 250. With the ratchet-gear mechanism 50 provided in this drill-guide assembly 5, the serrations 102 on the release knob 100 can be used to releasably lock Y-shaped ratchet-gear mechanism 50 at the desired level of actuation. This may obviate the need for a surgeon or user to continue to depress the trigger 56 relative to handle member 250 after desired actuation has occurred. The pawls 58 on the second leg 54 of the Y-shaped ratchet-gear mechanism 50 may engage the serrations 102 on the release knob 100 when the trigger 56 is pressed sufficiently. The release knob 100 may be held in a fixed position as a result of the compression force exerted by the compressed spring 272.

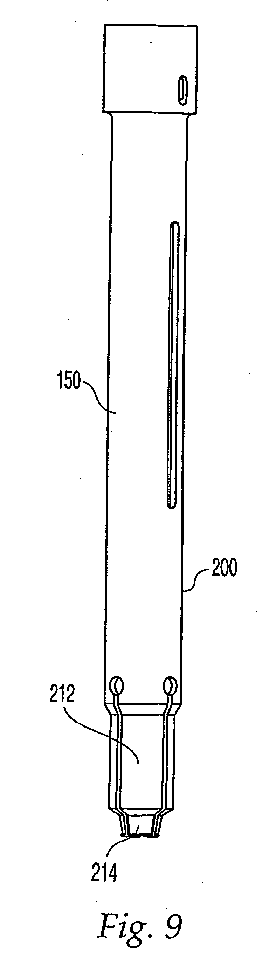

When the release knob 100 is pressed in the direction of the front end 254 of the handle member 250, the spring member 272 may be compressed, the pawls 58 may be disengaged from the serrations 102, and the Y-shaped ratchet-gear mechanism 50 may become unactuated. When the Y-shaped ratchet-gear mechanism 50 is unactuated, the force that is keeping the alignment drill-barrel 150 in a position toward fingers 210 may be released. As a result, the alignment drill-barrel 150 may no longer be pushing the fingers 210 on the bushing 200 in an outward direction toward the bone-plate 350. The alignment drill-barrel 150 can be then moved in a longitudinal direction away from the fingers 210 on the bushing 200. As a result, the bushing 200 may assume a retracted position as demonstrated in FIG. 9. Once the fingers 210 retract, the drill-assembly 5 may unlock from the fastener hole 352 or drill recess 354 of the bone-plate 350 and the user or surgeon can withdraw it.

When the release knob 100 is pressed to further compress the spring, the pawls 58 may disengage from serrations 102, thereby de-actuating the Y-shaped ratchet-gear mechanism 50, which in turn, through the pivot action at the pivot screw 64 may result in the movement of the alignment drill-barrel 150 in a direction away from the bone-plate 350.

Advantageously, a surgeon or user can operate drill-guide 5 with only one hand, due to the ergonomic positioning of trigger 56 and handle member 250. With the embodiment illustrated in FIG. 1, a user can attach the drill-guide by using a finger, such as an index finger, to engage and manipulate the tail 56 of the ratchet-gear mechanism 50, and while a second different finger, such as a thumb, to engage and manipulate the release knob 100.

When the alignment drill-barrel 150 is in the unlocked position as shown in FIG. 9, the conical section 184 allows fingers 210 to return to a relaxed, contracted position. This allows bushing 200 to be inserted and retracted from plate fastener hole. The inner surface of the bushing 200 forward of steps 220 in bushing 200 is preferably tapered at an angle θ_{B} to line 240 that is about 1 degree more than taper angle θ_{T} of conical sections 184, and preferably angle θ_{B} is about 4 degrees. A desirable amount of movement of alignment drill-barrel 150 within bushing 200 is thus provided to bias fingers 210 of bushing 200 from a contracted position to an expanded position. Alternative taper angles of conical section 184 and inner surface of bushing 200 may be chosen according to varying design criteria. In addition, a preferred, short movement of trigger 56 (ratchet-gear mechanism 50) is required to expand and contract fingers 210 of bushing 200.

Before and during bone plate implantation, the surgeon or user may insert the expandable distal end 222 of bushing 200 in particular neck 208 and rim 214, into fastener hole 352 or drill recess 354 in a bone plate 350. By pressing trigger 56 of the Y-shaped ratchet-gear mechanism 50 relative to the handle member 250, the surgeon or user may grasp and manipulate the plate 350 without an additional plate holder if so desired. Friction between the forward conical section 184 of the alignment drill-barrel 150 and the inner surface of fingers 210 especially at neck 208 and rim 214 may retain the expandable distal end 222 of bushing 200 in an expanded, locked position. Thus, when bushing 200 is in the expanded, locked position in a fastener hole of a plate placed in position for implantation, movement of the plate during the drilling operation can be minimized.

Drill-barrel 150 may preferably be sized so that once the bone plate 350 is properly positioned over the implantation site and bushing 206 is locked to the plate, the insertion point of a surgical drill-bit 400 at the proximal end of drill-barrel 150, is located at a distance beyond the patient's body such that a spinning surgical drill-bit 400 will not laterally reach or harm surrounding tissues that the surgeon or user does not intend to drill.

Preferably, the surgical drill-bits used with surgical drill-guide assembly 5 are configured and dimensioned to drill holes of about 12, 14, or 16 mm in depth. Suitable drill-bits typically have integral stops so that when the drill-bits are used with alignment drill-barrel of an established length, the holes produced by the drill-bit will not be deeper than the intended depth using a given bit. The stops may be positioned to abut the upper surfaces at the proximal end of drill-barrel 150, when a drill-bit has been inserted in the barrel to a particular depth.

Another embodiment of a drill-guide assembly 500 is shown in FIGS. 12A-13B. As with drill-guide assembly 5 (see FIG. 1, *supra*), assembly 500 may include an alignment assembly 515, release knob 600, handle member 650, ratchet-gear mechanism 550, bushing 450 with fingers 570 and slits 572, and a dual-arm support 610, the components of which may exhibit some or all of the characteristics of the corresponding components described above in relation to assembly 5.

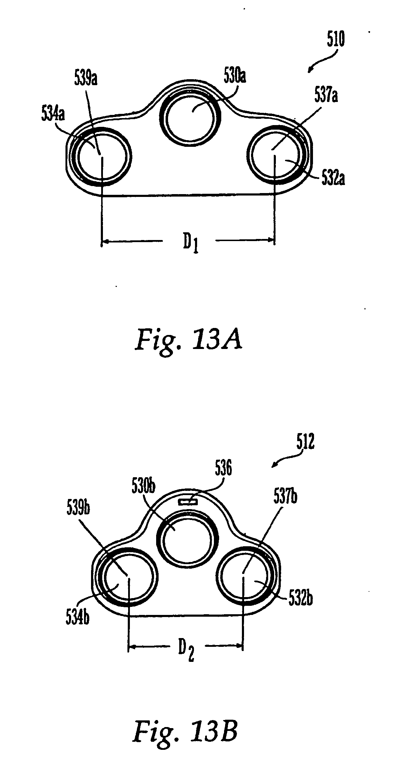

Drill-guide assembly 500 may also include first and second drill guides 502, 504 for use with surgical drill (e.g., 400). Drill guides 502, 504 may be connected to bushing 450 by proximal and distal connecting elements 510, 512, which are discussed in more detail below in relation to FIGS. 13A-13B. Drill guides 502, 504 may also have proximal ends 506, 508 and distal ends 516, 518, with a bores 507, 509 extending therebetween. The bores 507, 509 should be sized to receive at least a portion of a surgical drill, and should preferably align with a bone fastener hole 352 during use. Drill guides 502, 504 may have a length L₁, L₂ (*see* Figs. 12A-B) from about 150 mm to about 350 mm, and more preferably, a length of about 260 mm. Generally, drill guides 502, 504 have a greater length than bushing 450. Drill guide lengths L₁, L₂ may or may not be approximately equal.

Bores 507, 509 may have a variable diameter B₁, B₂ along the length L₁, L₂ of drill guides 502, 504. Bore diameter B₁, B₂ may have a diameter of about 5 mm to about 15 mm at proximal ends 506, 508 and/or distal ends 516, 518.

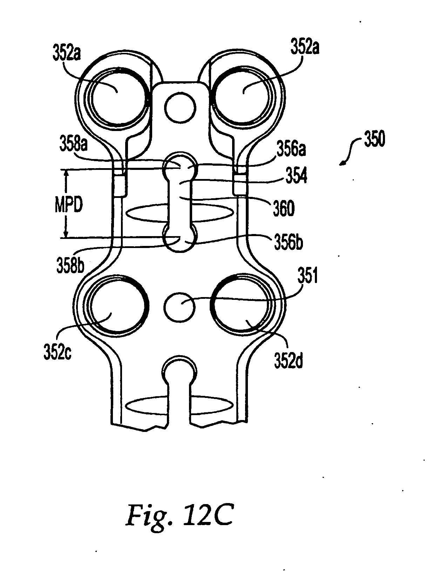

Drill-guide assembly 500 may be used with the plate shown in FIG. 12C. Plate 350 may have a plurality of fastener holes 352 and at least one drill recess 354 in body 351. Recess 354 may have shaped areas 356a, 356b with midpoint 358a, 358b, with a distance MPD between midpoints. Recess 354 may also have a slot area 360 extending between shaped areas 356a, 356b.

Alternatively, recess 354 may at least partially comprise a polygonal shape, such as a hexagon, rectangle, or square. The recess 354 may also take the shape of a plurality of polygonal shapes, for example, two overlapping hexagons may comprise the shape of the recess 354 to form a combination-polygonal recess. These embodiments may be particularly useful in bone-plates with a reduced area in which to place a recess 354 for purposes of aligning assembly 500.

In use, the fingers 570 of bushing 450 of assembly 500 may be inserted into drill recess 354, instead of fastener hole 352. The engagement and/or locking of the bushing 450 within a drill recess 354 may take some or all of the characteristics of the engagement and/or locking of bushing 200 with a fastener hole 352, as described above. Generally, it may be preferable for the bushing 450 to engage the drill recess 354 at shaped area 356a, 356b. The placement and locking of bushing 450 at shaped area 356a may align drill guides 502, 504 with fastener holes 352a, 352b, respectively. Similarly, the placement and locking of bushing 450 at shaped area 356b may align drill guides 502, 504 with fastener holes 352c, 352d, respectively.

Assembly 500 may also have a fin 514 to assist the insertion, locking, and/or alignment of the assembly in a drill recess 354. Fin 514 may generally be an elongated component, with at least a portion of the fin 514 secured in the distal connecting element 512 at fin bore 536 (see FIG 13B). In use, when the bushing engages a shaped area 356a, 356b, the fin concurrently engages slot 360. The fin 514 may or may not touch the sides of the slot 360 when the bushing 450 is fully inserted into a shaped area 356a, 356b.

FIG. 13A is a top view of a proximal connecting element 510, and FIG. 13B is a top view of a distal connecting element 512. Proximal connecting element 510 may have a bushing bore 530a, and first and second drill guide bores 532a, 534a. First and second drill guide bores 532a, 534a may have respective midpoints 537a, 539a, wherein a distance D₁ extends between midpoints 537a, 539a. Distal connecting element 512 similarly may have a bushing bore 530b, and first and second drill guide bores 532b, 534b with respective midpoints 537b, 539b. Midpoints 537b, 539b may have a distance D₂ between them. Distal connecting element 512 may also have a fin bore 536 located near the bushing bore 530b. Fin bore 536 may receive at least a portion of a fin 514, as discussed above.

Bushing bores 530a, 530b may receive at least a portion of a bushing 450. Likewise, first and second drill guide bores 532a, 534a, 532b, 534b may receive at least a portion of a first and second drill guide 502, 504, respectively. Generally, the proximal connecting element 510 may be situated near the proximal ends 506, 508 of first and second drill guide 502, 504, and the distal connecting element 512 may be situated near the distal ends 516, 518 of the first and second drill guides 502, 504. While the embodiment in FIGS. 12A-12B show two connecting elements, 510, 512 it contemplated that only one connecting element could be used, or that more than two connecting elements could be utilized with a single assembly 500.

The placement of the bores in the connecting elements 510, 512 may determine the angles and arrangements of which the bushing 450 and first and second drill guides 502, 504 are situated in relation to one another. For instance, the embodiment shown in FIGS 12A-13B utilizes proximal connecting element 510 with distance D₁ larger than the distance D₂ of the distal connecting element 512. The result of this arrangement is, as bushing 450 and first and second drill guides 502, 504 are generally linear, that the bushing and drill guides are generally convergent from the proximal end of the assembly to the distal end of the assembly 500. However, it is contemplated that D₁ and D₂ could be substantially equal, thereby creating an arrangement where the bushing and drill guides would be substantially parallel. Moreover, D₂ may be greater than D₁, thereby creating a divergent relationship between the bushing and/or drill guides from the proximal to the distal end of the assembly 500. Generally, both D₁ and D₂ may be from about 5 mm to about 35 mm. The sizes of the bores of each connecting element 510, 512 may generally fit a desired engagement portion of a bushing and/or drill guide.

Those skilled in the art will recognize that bushing 200, 450 may be configured and dimensioned to fit bone plate fastener holes and/or drill recesses with shapes other than circular. For example, bushing 200, 450 may be adapted to fit elliptical, hexagonal, star-shaped, or square fastener holes and/or drill recesses.

Preferably, the components of surgical drill-guide assembly 5 are metallic, passivated, and electropolished. Most preferably, the components are formed of stainless steel, except for the springs which are formed of spring steel, although other materials may be used. Preferably, at least the handle member is forged, while the other components may be machined, and the surgical drill-guide assembly preferably has a matte finish so that the surfaces of the components do not reflect operating room light in such a manner as to distract the surgeon or user. Some components may be subjected to heat treatments so that the surfaces are work hardened. The surfaces are preferably burr-free. Preferably, the surface finish allows individual components to move with respect to each other in a smooth and non-binding fashion through each component's entire range of motion. Additionally, all pins and fasteners are preferably flush with the surfaces into which they are fixed.

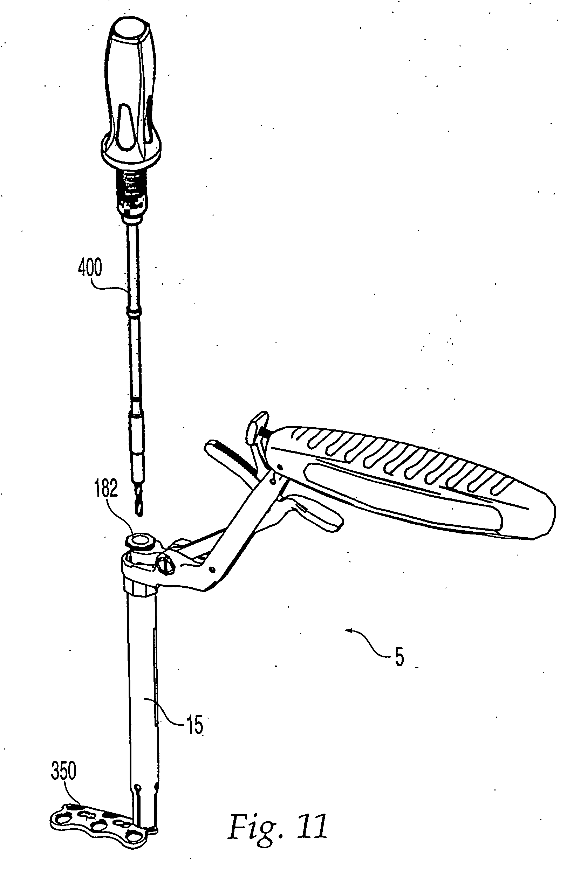

The present invention can be used in several methods of drilling holes. In one method, a surgeon or user may insert the bushing of a surgical drill-guide assembly into a fastener hole of a bone-plate and may depress the ratchet-gear mechanism to slide the alignment drill-barrel forward, expanding the bushing preferably by the conical portions of the alignment drill-barrel radially spreading the fingers in the bushing. The surgeon or user may then lock the bushing to the plate by locking the alignment drill-barrel and the bushing in fixed relation to each other, which thereby may relieve the surgeon or user of the need to squeeze the ratchet-gear mechanism toward the handle (see FIG. 11). The surgeon or user may align the surgical drill-bit along the drilling axis defined through the center of the bore in the alignment drill-barrel and inserts the drill-bit in the barrel. The surgeon or user then may drill a first hole coaxial with the central axis of a first fastener hole in the plate. The drill-bit may be stopped at a predetermined distance to provide a hole of predetermined depth. The drill-bit may be removed from the alignment drill-barrel. The bushing may thereafter be unlocked from the plate by pressing the release knob, which may release the bushing from the fastener hole so that the user can then freely and unfetteredly remove the drill-guide assembly from the plate.

In another use, a surgeon or user may insert the bushing of a surgical drill-guide assembly into a shaped area drill recess of a bone-plate and may depress the ratchet-gear mechanism to slide the alignment drill-barrel forward, expanding the bushing preferably by the conical portions of the alignment drill-barrel radially spreading the fingers in the bushing. The fin of the assembly may concurrently engage the slot of the recess. The surgeon or user may then lock the bushing to the plate by locking the alignment drill-barrel and the bushing in fixed relation to each other, which thereby may relieve the surgeon or user of the need to squeeze the ratchet-gear mechanism toward the handle (see FIG. 11). The surgeon or user may align the surgical drill-bit along the drilling axis defined through the centers of the bores of the first and/or second drill guides and may insert the drill-bit into the bores as desired.

While the invention has been shown and described herein with reference to particular embodiments, it is to be understood that the various additions, substitutions, or modifications of form, structure, arrangement, proportions, materials, and components and otherwise, used in the practice of the invention and which are particularly adapted to specific environments and operative requirements, may be made to the described embodiments without departing from the scope of the present invention. For example, the surgical drill-guide assembly may have alignment drill-barrel that can be angulated in the cephalad/caudal or sagittal planes, thereby permitting a range of angles to be chosen for the holes to be drilled and further permitting a range of spacings of plate holes to be accommodated. Moreover, alignment drill-barrel that is removeably attachable to the base may be provided so that a surgeon or user may select alignment drill-barrel with holes that precisely accommodate a desired drill-bit size. In addition, the drill-guide assembly handle may include a grip that generally follows the contours of fingers that hold the grip. The presently disclosed embodiments are therefore to be considered in all respects as illustrative and not restrictive, the scope of the invention being indicated by the appended claims, and not limited to the foregoing description.
The following methods of using the claimed drill-guide assembly are also described herein.
A first method for drilling holes in bone, comprising the step of :
(a) providing a drill-guide assembly having an alignment barrel configured to receive and guide a drill-bit; a bushing configured to slidably receive the alignment barrel, the bushing having a radially expandable forward-end configured to be insertable within a hole or recess in a bone plate; a handle member for grasping by a user; a dual-arm support having first and second parts, the first part of the dual-arm support fixedly connected to the proximal end of the bushing and the second part of the dual-arm support fixedly connected to the handle member; a release knob moveably connected to the handle member, the release knob having serrations; and a ratchet gear mechanism having a first leg, a second leg and a tail, the first leg of the ratchet-gear mechanism pivotably connected to the dual-arm support and further connected to the alignment barrel, the second leg of the ratchet-gear mechanism having pawls that engage the serrations when the ratchet gear mechanism selectively moves, the tail of the ratchet gear mechanism operable by a user to selectively move the ratchet-gear mechanism, wherein, movement of the ratchet-gear mechanism slides the alignment barrel relative to the bushing to radially expand the forward end to releasably lock the bushing to the plate;
(b) inserting the bushing of a drill-guide assembly into fastener holes of a bone-plate;
(c) expanding the bushing in the fastener hole;
(d) locking the bushing to the plate by locking the alignment barrel and the bushing in fixed relation to each other;
(e) aligning a drill-bit along the drilling axis defined through the center of the bore in the alignment barrel;
(f) drilling a first hole;
(g) stopping the drill-bit at a predetermined distance to provide a hole of predetermined depth; and
(h) removing the drill-bit from the alignment barrel.
A second method for drilling holes in bone, according to said first method, further comprising the steps of unlocking the bushing from the plate by pressing the release knob, and releasing the bushing from the fastener hole so that the user can remove the drill-guide assembly from the plate.
A third method for drilling holes in bone, comprising the steps of:
(a) providing a drill-guide assembly, comprising an alignment barrel having a proximal end and a distal end; a bushing configured to slidably receive the alignment barrel, the bushing having a radially expandable forward-end and a proximal end, the forward-end configured to be insertable within a hole or recess in a bone plate; a release knob having serrations; and a movable ratchet gear mechanism having a first leg, a second leg and a tail, the first leg of the ratchet-gear mechanism connected to the alignment barrel, the second leg of the ratchet-gear mechanism having pawls configured and adapted to engage the serrations to hold the alignment barrel in position, the tail of the ratchet gear mechanism operable by a user to selectively move the ratchet-gear mechanism, wherein, movement of the ratchet-gear mechanism slides the alignment barrel relative to the bushing to radially expand the forward end to releasably lock the bushing to the plate, and at least a first guide coupled to the bushing, wherein the first drill guide is configured to receive and guide a drill-bit;
(b) inserting the bushing into a recess of a bone plate;
(c) aligning at least the first drill guide with a first bone fastener hole in the bone-plate;
(d) expanding the bushing in the recess;
(e) locking the bushing to the plate;
(f) inserting a drill-bit into the first drill guide; and
(g) drilling a first hole.
The third method, wherein at least a portion of the forward end of the bushing is configured to fit in at least a portion of the recess.
The third method, wherein the bushing is locked to the plate by locking the alignment barrel and bushing in fixed relation to each other.
The third method, wherein the drill guide assembly further comprising a second drill guide coupled to the bushing, wherein the second drill guide is configured to receive and guide a drill-bit.
The above method, further comprising the steps of inserting a drill-bit guide into the second drill guide, and drilling a second hole.

## Claims

1. A drill-guide assembly (5; 500), comprising:
an alignment barrel (150) having a proximal end (174) and a distal end (172);
a bushing (200; 450) configured to slidably receive the alignment barrel (150), the bushing (200; 450) having a radially expandable forward-end (222) and a proximal end, the forward-end (222) configured to be insertable within a hole (352) or recess (354) in a bone plate (350);
a release knob (100); and
a movable gear mechanism (50; 550) having a first leg (52), a second leg (54) and a tail (56),
the first leg (52) of the gear mechanism (50; 550) connected to the alignment barrel (150),
the second leg (54) of the gear mechanism (50; 550) adapted to hold the alignment barrel (150) in position,
the tail (56) of the gear mechanism (50; 550) operable by a user to selectively move the gear mechanism (50; 550),
wherein movement of the gear mechanism (50; 550) slides the alignment barrel (150) relative to the bushing (200; 450) to radially expand the forward end (222) to releasably lock the bushing (200; 450) to the plate (350),
**characterized in that** the movable gear mechanism (50; 550) is a movable ratchet-gear mechanism, and **in that** the release knob (100; 600) has serrations (102), and the second leg (54) of the ratchet-gear mechanism (50; 550) has pawls (58) configured and adapted to engage the serrations (102) to hold the alignment barrel (150) in position.

2. The drill-guide assembly, as recited in claim 1, wherein the ratchet-gear mechanism (50; 550) is pivotably mounted, or
wherein the release knob (100) is pivotably mounted, or
wherein the ratchet-gear mechanism (50; 550) swivels incrementally, or
wherein the alignment barrel (150) comprises a through bore (185) from the distal end (172) to the proximal end (174).

3. The drill-guide assembly, as recited in claim 1 or 2, wherein the alignment drill barrel (150) further comprises a first hollow cylindrical section (156), a second hollow cylindrical section (158) and a third hollow cylindrical section (160) wherein the annular diameter (X₂₄) of the third cylindrical section (160) is at least equal to the annular diameter (X₁₈) of the second cylindrical section (158), and wherein the annular diameter (X₁₈) of the second cylindrical section (158) is at least equal to the annular diameter (X₁₂) of the first cylindrical section (156), or
wherein the annular diameter (X₂₄) of the third cylindrical section (160) is constant along the center line (180) of the cylindrical section (160).

4. The drill-guide assembly as recited in any one of claims 1 to 3, wherein the alignment barrel (150) further comprises two ridges (152, 154) at the proximal end (174),
wherein preferably the outside surface of the alignment drill barrel (150) has a shoulder (164) at the distal end (172), or
wherein preferably the outside diameter (X₂₆) of the third cylindrical section (160) tapers to form a conical shape.

5. The drill-guide assembly, as recited in any one of claims 1 to 4, wherein the bushing (200; 450) further comprises radially expandable fingers (210; 570).

6. The drill-guide assembly, as recited in claim 5, wherein the fingers (210; 570) form a radially expandable circumferential neck (208) and assume an inward unexpanded disposition in relaxed state,
wherein preferably the bushing (200; 450) further comprises a shoulder (212) adjacent to the radially expandable circumferential neck (208).

7. The drill-guide assembly, as recited in claim 5 or 6, wherein the distal end (222) of the bushing (200; 450) comprises a tapered end with the inner and the outer diameter of the tapered end decreasing in direction of the tip, or
wherein the bushing (200; 450) is made from a single piece of material of unitary construction, or
wherein the bushing (200; 450) further comprises a pin for securing the drill-guide assembly (5; 500) to a bone-plate (350), or
wherein the bushing (200; 450) comprises of at least one vertical slot above the circular portion (206) on the bushing (200; 450).

8. The drill-guide assembly of any one of claims 1 to 7, wherein the alignment barrel (150) is configured to receive and guide a drill bit (400), or
further comprising at least a first drill guide (502) coupled to the bushing (200; 450), wherein at least the first drill guide (502) is configured to receive and guide a drill bit (400).

9. The drill-guide assembly (5; 500), as recited in any one of claims 1 to 8, further comprising:
a handle member (250; 650) for grasping by a user; and
a dual-arm support (10; 610) having first and second parts (14, 16), the first part (14) of the dual-arm support (10; 610) fixedly connected to the proximal end (222) of the bushing (200; 450) and the second part (16) of the dual-arm support (10; 610) fixedly connected to the handle member (250; 650);
wherein the release knob (100) is moveably connected to the handle member (250; 650); and
the first leg (52) of the ratchet-gear mechanism (50; 550) is pivotably connected to the dual-arm support (10; 610) and further connected to the alignment barrel (150).

10. The drill-guide assembly, as recited in claim 9, wherein the dual-arm support (10; 610) is L-shaped, or
wherein the dual-arm support (10; 610) is fixed to the bushing (200; 450) by a method of fixing selected from the group consisting of welding, friction fitting, and press fitting, or
wherein the first part (14) of the dual-arm support (10; 610) and the axial direction of the alignment assembly (15; 515) form an angle in the range of from about 75 degree to about 120 degree, or
wherein the second part (16) of the dual-arm support (10; 610) and the first part (14) of the dual-arm support (10; 610) form an angle in the range of from about 90 degree to about 150 degree, or
wherein the dual-arm support (10; 610) is an integral, monolithic construction, or
wherein the second part (16) of the dual-arm support (10; 610) and the handle member (250; 650) form an angle in the range from about 90 degree to about 150 degree, or
wherein the longitudinal axis (280) of the handle member (250; 650) lies in the same plane as the longitudinal axis (24) of the first part (14) of the dual-arm support (10; 610), and the longitudinal axis (26) of the second part (16) of the dual-arm support (10; 610), or
wherein the longitudinal axis (280) of the handle member (250; 650) is parallel to the longitudinal axis (24) of the first part (14) of the dual-arm support (10; 610), or
wherein the dual-arm support (10; 610) is immovably connected to the bushing (200; 450) at the proximal end (242) of the bushing (200; 450) and the dual-arm support (10; 610) is immovably connected to the handle member (250; 650), or
wherein the dual-arm support (10; 610) has an open space and the ratchet-gear mechanism (50; 550) is disposed in the open space and pivotally mounted therein.

11. The drill-guide assembly, as recited in claim 9 or 10, wherein the handle member (250; 650) has a front end (254) and a back end, and wherein the dual-arm support (10; 610) and the handle member (250; 650) are fixedly connected at the front end (254) of the handle member (250; 650).

12. The drill-guide assembly, as recited in any one of claims 9 to 11, wherein the ratchet-gear mechanism (50; 550) is Y-shaped,
wherein preferably the first leg (52) of the ratchet-gear mechanism (50; 550) further extends beyond the pivot point forming a C-shaped vice-grip (60); wherein the C-shaped vice-grip (60) grasps the alignment barrel (150) between the two ridges (152, 154) on the alignment barrel (150), and
wherein further preferably the plane of the C-shaped vice-grip (60) makes an acute angle with the longitudinal axis (64) of the first leg (52) of the Y-shaped ratchet-gear mechanism (50; 550), or
wherein further preferably the acute angle is in a range of from about 25 degree to about 45 degree.

13. The drill-guide assembly, as recited in any one of claims 9 to 12, wherein the handle member (250; 650) is fixed to the dual-arm support (10; 610) with a pin (20) that is perpendicular to the axis (280) of the handle member (250; 650), or
wherein said expandable forward-end (222) of said bushing (200; 450) is circular shaped, and freely insertable and removable from said hole (352) or recess (354) in a contracted position, and engaging the bone-plate (350) when in an expanded position.

14. The drill-guide assembly as recited in any one of claims 9 to 13, wherein said radially expandable forward-end (222) comprises a plurality of finger portions (210; 570).

15. The drill-guide assembly as recited in claim 14, wherein:
said radially expandable forward-end (222) of the bushing (200; 450) comprises a shoulder (212), a neck (208), and an outwardly projecting rim (214) disposed forward of said neck (208);
wherein said neck (208) and rim (214) together span a length that is slightly longer than the thickness of the hole (352) or recess (354) wall, and the rim (214) abuts the bone-side surface of said plate (350).

16. The drill-guide assembly, as recited in claim 15, wherein the handle member (250; 650) comprises a first cavity (256) and a second cavity (258) at the front end (254), wherein the first cavity (526) houses a compression spring (272), and the second cavity (258) houses the second part (16) of the dual-arm support (10; 610).

17. The drill-guide assembly, as recited in any one of claims 9 to 16, wherein the alignment barrel (150) is configured to receive and guide a drill bit (400), or
further comprising at least a first drill guide (502) coupled to the bushing (200; 450), wherein at least the first drill guide (502) is configured to receive and guide a drill bit (400).

18. The drill-guide assembly, as recited in any one of claims 1 to 17, further comprising:
a first drill guide (502) coupled to the bushing (200; 450), wherein the first drill guide (502) is configured to receive and guide a drill-bit (400).

19. The drill-guide assembly of claim 18, wherein the first drill guide (502) is coupled to the bushing (200; 450) by a first connecting element (510).

20. The drill-guide assembly of claim 19, wherein the first connecting element (510) has at least two bores (530a, 532a, 534a) for respectively receiving at least a portion a bushing (200; 450) therethrough and at least a portion of a drill guide (502, 504) therethrough.

21. The drill-guide assembly of claim 20, wherein the first drill guide (502) is further coupled to the bushing (200; 450) by a second connecting element (512),
wherein preferably the second connecting element (512) has at least two bores (530b, 532b, 534b) for respectively receiving at least a portion of a bushing (200; 450) therethrough and at least a portion of a drill guide (502, 504) therethrough.

22. The drill-guide assembly of claim 21, wherein the at least two bores (530a, 532a, 534a) of the first connecting element (510) are separated by a first distance (D₁), and the at least two bores (530b, 532b, 534b) of the second connecting element (512) are separated by a second distance (D₂), wherein the first distance (D₁) is greater than the second distance (D₂), and wherein the second connecting element (512) is closer to the distal end of the forward end (222) of the bushing (200; 450) than the first connecting element (510).

23. The drill-guide assembly of claim 21, wherein the at least two bores (530a, 532a, 534a) of the first connecting element (510) are separated by a first distance (D₁), and the at least two bores (530b, 532b, 534b) of the second connecting element (512) are separated by a second distance (D₂), wherein the first distance (D₁) is greater than the second distance (D₂), and wherein the first connecting element (510) is closer to the distal end of the forward end (222) of the bushing (200; 450) than the second connecting element (512).

24. The drill-guide assembly of claim 20, wherein the first connecting element (510) further comprises a fin bore (536) configured to receive at least a portion of fin (514) therethrough, wherein at least a portion of the fin (514) is configured to engage at least a portion of a hole (352) or recess (354) when the bushing (200; 450) engages a bone-plate (350).

25. The drill-guide assembly of any one of claims 18 to 24, further comprising a second drill guide (504) coupled to the bushing (200; 450), wherein the second drill guide (504) is configured to receive and guide a drill-bit (400),
wherein preferably the second drill guide (504) is coupled to the first drill guide (502), and
wherein further preferably the first and second drill guides (502, 504) are coupled to the bushing (200; 450) by a first connecting element (510), and
wherein still further preferably the first connecting element (510) has at least three bores (530a, 532a, 534a) for respectively receiving at least a portion of the first drill guide (502) therethrough, at least a portion of the second drill guide (504) therethrough, and a least a portion of the bushing (200; 450) therethrough.

26. The drill-guide assembly of claim 25, wherein the first connecting element (510) further comprises a fin bore (536) configured to receive at least a portion of fin (514) therethrough, and wherein at least a portion of the fin (514) is configured to engage at least a portion of a hole (352) or recess (354) when the bushing (200; 450) engages a bone-plate (350), or
wherein the first drill guide (502) and second drill guide (504) are further coupled to the bushing (200; 450) by a second connecting element (512).

27. The drill-guide assembly of claim 26, wherein the second connecting element (512) has at least three bores (530b, 532b, 534b) for respectively receiving at least a portion of the first drill guide (502) therethrough, at least a portion of the second drill guide (504) therethrough, and a least a portion of the bushing (200; 450) therethrough.

28. The drill-guide assembly of claim 27, wherein the bores (530a, 532a, 534a) of the first connecting element (510) receiving first and second drill guides (502, 504) are separated by a first distance (D₁), and the bores (530b, 532b, 534b) of the second connecting element (512) receiving first and second drill guides (502, 504) are separated by a second distance (D₂), wherein the first distance (D₁) is greater than the second distance (D₂), and wherein the second connecting element (512) is closer to the distal end of the forward end (222) of the bushing (200; 450) than the first connecting element (510).

29. The drill-guide assembly of claim 27, wherein the bores (530a, 532a, 534a) of the first connecting element (510) receiving first and second drill guides (502, 504) are separated by a first distance (D₁), and the bores (530b, 532b, 534b) of the second connecting element (512) receiving first and second drill guides (502, 504) are separated by a second distance (D₂), wherein the first distance (D₁) is greater than the second distance (D₂), and wherein the first connecting element (510) is closer to the distal end of the forward end (222) of the bushing (200; 450) than the second connecting element (512).

30. The drill-guide assembly of any one of claims 18 to 29, the first drill guide (502) having a longitudinal axis, and wherein when the bushing (200; 450) is locked to a bone-plate (350), the longitudinal axis of the first drill guide (502) is generally aligned with a first bone-fastener hole (352) of the bone plate (350).

31. The drill-guide assembly of claim 30, further comprising a second drill guide (504) configured to receive and guide a drill-bit (400) and coupled to the bushing (200; 450), the second drill guide (504) having a longitudinal axis, and wherein when the bushing (200; 450) is locked to a bone-plate (350), the longitudinal axis of the second drill guide (504) is generally aligned with a second bone-fastener hole (352) of the bone-plate (350).

32. The drill-guide assembly of any one of claims 18 to 31, wherein the recess (354) of the bone-plate (350) includes at least one shaped area (356) and a slot (360).

33. A kit for use with drilling bones, comprising:
(a) a drill-guide assembly (5; 500), as recited in any one of claims 1 to 32;
(b) at least first and second drill guides (502, 504) able to be coupled to the bushing (200; 450); and
(c) at least first and second connecting elements (510, 512) for coupling at least one drill guide (502, 504) to the bushing (200; 450).

34. The kit of claim 33, wherein at least the first and second drill guides (502, 504) have different lengths, or
wherein at least the first and second drill guides (502, 504) have different diameters.

35. The kit of claim 33 or 34, wherein at least the first and second connecting elements (510, 512) each have a bore (532, 534) for receiving at least one drill guide (502, 504) therethrough and a bore (530) for receiving a bushing (200; 450) therethrough, wherein the bores (530a, 532a, 534a) of the first connecting element (510) have a first arrangement, and the bores (530b, 532b, 534b) of the second connecting element (512) have a second arrangement, and wherein the first arrangement is substantially different than the second arrangement.

## Patentansprüche

1. Bohrerführungsanordnung (5; 500), umfassend:
ein Ausrichtungsrohr (150), welches ein proximales Ende (174) und ein distales Ende (172) aufweist;
eine Buchse (200; 450), welche eingerichtet ist, das Ausrichtungsrohr (150) gleitend aufzunehmen, wobei die Buchse (200; 450) ein radial expandierbares Vorwärtsende (222) und ein proximales Ende aufweist, wobei das Vorwärtsende (222) eingerichtet ist, in ein Loch (352) oder eine Ausnehmung (354) in einer Knochenplatte (350) einführbar zu sein;
einen Freigabeknopf (100); und
einen beweglichen Gangmechanismus (50; 550), welcher einen ersten Schenkel (52), einen zweiten Schenkel (54) und einen Ausläufer (56) aufweist,
wobei der erste Schenkel (52) des Gangmechanismus (50; 550) mit dem Ausrichtungsrohr (150) verbunden ist,
wobei der zweite Schenkel (54) des Gangmechanismus (50; 550) angepasst ist, das Ausrichtungsrohr (150) in Position zu halten,
wobei der Ausläufer (56) des Gangmechanismus (50; 550) durch einen Benutzer bedienbar ist, um den Gangmechanismus (50; 550) selektiv zu bewegen,
wobei eine Bewegung des Gangmechanismus (50; 550) das Ausrichtungsrohr (150) relativ zur Buchse (200; 450) verschiebt, um das Vorwärtsende (222) radial zu expandieren, um die Buchse (200; 450) lösbar an der Platte (350) zu verriegeln,
**dadurch gekennzeichnet, dass** der bewegliche Gangmechanismus (50; 550) ein beweglicher Ratschen-Gangmechanismus ist, und dadurch, dass der Freigabeknopf (100; 600) eine Verzahnung (102) aufweist, und der zweite Schenkel (54) des Ratschen-Gangmechanismus (50; 550) Klauen (58) aufweist, welche eingerichtet und angepasst sind, mit der Verzahnung (102) zusammenzuwirken, um das Ausrichtungsrohr (150) in Position zu halten.

2. Bohrerführungsanordnung nach Anspruch 1, wobei der Ratschen-Gangmechanismus (50; 550) schwenkbar montiert ist, der
wobei der Freigabeknopf (100) schwenkbar montiert ist, oder
wobei der Ratschen-Gangmechanismus (50; 550) inkrementell schwenkt, oder
wobei das Ausrichtungsrohr (150) eine Durchgangsbohrung (185) vom distalen Ende (172) zum proximalen Ende (174) umfasst.

3. Bohrerführungsanordnung nach Anspruch 1 oder 2, wobei das Ausrichtungsbohrrohr (150) des Weiteren einen ersten hohlen zylindrischen Abschnitt (156), einen zweiten hohlen zylindrischen Abschnitt (158) und einen dritten hohlen zylindrischen Abschnitt (160) umfasst, wobei der Ringdurchmesser (X₂₄) des dritten zylindrischen Abschnitts (160) zumindest gleich dem Ringdurchmesser (X₁₈) des zweiten zylindrischen Abschnitts (158) ist, und wobei der Ringdurchmesser (X₁₈) des zweiten zylindrischen Abschnitts (158) zumindest gleich dem Ringdurchmesser (X₁₂) des ersten zylindrischen Abschnitts (156) ist, oder
wobei der Ringdurchmesser (X₂₄) des dritten zylindrischen Abschnitts (160) entlang der Mittellinie (180) des zylindrischen Abschnitts (160) konstant ist.

4. Bohrerführungsanordnung nach einem der Ansprüche 1 bis 3, wobei das Ausrichtungsrohr (150) des Weiteren zwei Rippen (152, 154) am proximalen Ende (174) umfasst,
wobei vorzugsweise die äußere Oberfläche des Ausrichtungsbohrrohrs (150) eine Schulter (164) am distalen Ende (172) aufweist, oder
wobei sich der äußere Durchmesser (X₂₆) des dritten zylindrischen Abschnitts (160) vorzugsweise verjüngt, um eine konische Form zu bilden.

5. Bohrerführungsanordnung nach einem der Ansprüche 1 bis 4, wobei die Buchse (200; 450) des Weiteren radial expandierbare Finger (210; 570) umfasst.

6. Bohrerführungsanordnung nach Anspruch 5, wobei die Finger (210; 570) einen radial expandierbaren umlaufenden Kragen (208) bilden und im entspannten Zustand eine nach innen unexpandierte Anordnung annehmen,
wobei die Buchse (200; 450) vorzugsweise des Weiteren eine Schulter (212) benachbart zu dem radial expandierbaren umlaufenden Kragen (208) umfasst.

7. Bohrerführungsanordnung nach Anspruch 5 oder 6, wobei das distale Ende (222) der Buchse (200; 450) ein sich verjüngendes Ende umfasst, wobei der innere und der äußere Durchmesser des sich verjüngenden Endes in Richtung der Spitze abnehmen, oder
wobei die Buchse (200; 450) aus einem einzigen Stück Material mit einheitlichem Aufbau hergestellt ist, oder
wobei die Buchse (200; 450) des Weiteren einen Stift zum Sichern der Bohrerführungsanordnung (5; 500) an einer Knochenplatte (350) umfasst, oder
wobei die Buchse (200; 450) zumindest einen vertikalen Schlitz über dem kreisförmigen Teil (206) auf der Buchse (200; 450) umfasst.

8. Bohrerführungsanordnung nach einem der Ansprüche 1 bis 7, wobei das Ausrichtungsrohr (150) eingerichtet ist, einen Bohrer (400) aufzunehmen und zu führen, oder
des Weiteren umfassend zumindest eine erste Bohrerführung (502), welche an die Buchse (200; 450) gekoppelt ist, wobei zumindest die erste Bohrerführung (502) eingerichtet ist, einen Bohrer (400) aufzunehmen und zu führen.

9. Bohrerführungsanordnung (5; 500) nach einem der Ansprüche 1 bis 8, des Weiteren umfassend:
ein Griffelement (250; 650) zum Greifen durch einen Benutzer; und
einen Zwei-Arm-Träger (10; 610), welcher einen ersten und zweiten Teil (14, 16) aufweist, wobei der erste Teil (14) des Zwei-Arm-Trägers (10; 610) fest mit dem proximalen Ende (222) der Buchse (200; 450) verbunden ist und der zweite Teil (16) des Zwei-Arm-Trägers (10; 610) fest mit dem Griffelement (250; 650) verbunden ist;
wobei der Freigabeknopf (100) beweglich mit dem Griffelement (250; 650) verbunden ist; und
der erste Schenkel (52) des Ratschen-Gangmechanismus (50; 550) schwenkbar mit dem Zwei-Arm-Träger (10; 610) verbunden ist und des Weiteren mit dem Ausrichtungsrohr (150) verbunden ist.

10. Bohrerführungsanordnung nach Anspruch 9, wobei der Zwei-Arm-Träger (10; 610) L-förmig ist, oder
wobei der Zwei-Arm-Träger (10; 610) an der Buchse (200; 450) fixiert ist mittels eines Fixierverfahrens ausgewählt aus der Gruppe bestehend aus Schweißen, Kraftschluss, und Formschluss, oder
wobei der erste Teil (14) des Zwei-Arm-Trägers (10; 610) und die axiale Richtung der Ausrichtungsanordnung (15; 515) einen Winkel im Bereich von ungefähr 75 Grad bis ungefähr 120 Grad bilden, oder
wobei der zweite Teil (16) des Zwei-Arm-Trägers (10; 610) und der erste Teil (14) des Zwei-Arm-Trägers (10; 610) einen Winkel im Bereich von ungefähr 90 Grad bis ungefähr 150 Grad bilden, oder
wobei der Zwei-Arm-Träger (10; 610) eine einstückige, monolithische Konstruktion ist, oder
wobei der zweite Teil (16) des Zwei-Arm-Träger (10; 610) und das Griffelement (250; 650) einen Winkel im Bereich von ungefähr 90 Grad bis ungefähr 150 Grad bilden, oder
wobei die Längsachse (280) des Griffelements (250; 650) in derselben Ebene wie die Längsachse (24) des ersten Teils (14) des Zwei-Arm-Trägers (10; 610) und die Längsachse (26) des zweiten Teils (16) des Zwei-Arm-Trägers (10; 610) liegt, oder
wobei die Längsachse (280) des Griffelements (250; 650) parallel zur Längsachse (24) des ersten Teils (14) des Zwei-Arm-Trägers (10; 610) ist, oder
wobei der Zwei-Arm-Träger (10; 610) am proximalen Ende (242) der Buchse (200; 450) unbeweglich mit der Buchse (200; 450) verbunden ist und der Zwei-Arm-Träger (10; 610) unbeweglich mit dem Griffelement (250; 650) verbunden ist, oder
wobei der Zwei-Arm-Träger (10; 610) einen offenen Raum aufweist und der Ratschen-Gangmechanismus (50; 550) in dem offenen Raum angeordnet ist und schwenkbar darin montiert ist.

11. Bohrerführungsanordnung nach Anspruch 9 oder 10, wobei das Griffelement (250; 650) ein vorderes Ende (254) und ein hinteres Ende aufweist, und wobei der Zwei-Arm-Träger (10; 610) und das Griffelement (250; 650) an dem vorderen Ende (254) des Griffelements (250; 650) fest verbunden sind.

12. Bohrerführungsanordnung nach einem der Ansprüche 9 bis 11, wobei der Ratschen-Gangmechanismus (50; 550) Y-förmig ist,
wobei vorzugsweise der erste Schenkel (52) des Ratschen-Gangmechanismus (50; 550) sich weiter über den Schwenkpunkt hinaus erstreckt und eine C-förmige Klemmzange (60) bildet; wobei die C-förmige Klemmzange (60) das Ausrichtungsrohr (150) zwischen den zwei Rippen (152, 154) auf dem Ausrichtungsrohr (150) greift, und
wobei weiter vorzugsweise die Ebene der C-förmigen Klemmzange (60) einen spitzen Winkel mit der Längsachse (64) des ersten Schenkels (52) des Y-förmigen Ratschen-Gangmechanismus (50; 550) bildet, oder
wobei weiter vorzugsweise der spitze Winkel in einem Bereich von ungefähr 25 Grad bis ungefähr 45 Grad liegt.

13. Bohrerführungsanordnung nach einem der Ansprüche 9 bis 12, wobei das Griffelement (250; 650) an dem Zwei-Arm-Träger (10; 610) mit einem Stift (20) fixiert ist, der senkrecht zu der Achse (280) des Griffelements (250; 650) ist, oder
wobei das expandierbare Vorwärtsende (222) der Buchse (200; 450) kreisförmig geformt ist, und frei einführbar und entfernbar aus dem Loch (352) oder der Ausnehmung (354) in einer zurückgezogenen Position ist, und mit der Knochenplatte zusammenwirkt (350), wenn in einer expandierten Position.

14. Bohrerführungsanordnung nach einem der Ansprüche 9 bis 13, wobei das radial expandierbare Vorwärtsende (222) eine Mehrzahl von Fingerteilen (210; 570) umfasst.

15. Bohrerführungsanordnung nach Anspruch 14, wobei:
das radial expandierbare Vorwärtsende (222) der Buchse (200; 450) eine Schulter (212), einen Kragen (208), und einen nach außen abstehenden Rand (214), der vor dem Kragen (208) angeordnet ist, umfasst;
wobei der Kragen (208) und der Rand (214) zusammen eine Länge aufspannen, die ein bisschen länger ist als die Dicke der Loch- (352) oder Ausnehmungs (354) - Wand, und der Rand (214) an die knochenseitige Oberfläche der Platte (350) angrenzt.

16. Bohrerführungsanordnung nach Anspruch 15, wobei das Griffelement (250; 650) eine erste Kavität (256) und eine zweite Kavität (258) am vorderen Ende (254) umfasst, wobei die erste Kavität (526) eine Kompressionsfeder (272) beherbergt und die zweite Kavität (258) den zweiten Teil (16) des Zwei-Arm-Trägers (10; 610) beherbergt.

17. Bohrerführungsanordnung nach einem der Ansprüche 9 bis 16, wobei das Ausrichtungsrohr (150) eingerichtet ist, einen Bohrer (400) aufzunehmen und zu führen, oder
des Weiteren umfassend zumindest eine erste Bohrerführung (502), welche an die Buchse (200; 450) gekoppelt ist, wobei zumindest die erste Bohrerführung (502) eingerichtet ist, einen Bohrer (400) aufzunehmen und zu führen.

18. Bohrerführungsanordnung nach einem der Ansprüche 1 bis 17, des Weiteren umfassend:
eine erste Bohrerführung (502), welche an die Buchse (200; 450) gekoppelt ist, wobei die erste Bohrerführung (502) eingerichtet ist, einen Bohrer (400) aufzunehmen und zu führen.

19. Bohrerführungsanordnung nach Anspruch 18, wobei die erste Bohrerführung (502) an die Buchse (200; 450) mittels eines ersten Verbindungselements (510) gekoppelt ist.

20. Bohrerführungsanordnung nach Anspruch 19, wobei das erste Verbindungselement (510) zumindest zwei Bohrungen (530a, 532a, 534a) zum entsprechenden Aufnehmen zumindest eines Teils einer Buchse (200; 450) dahindurch und zumindest eines Teils einer Bohrerführung (502, 504) dahindurch aufweist.

21. Bohrerführungsanordnung nach Anspruch 20, wobei die erste Bohrerführung (502) des Weiteren mittels eines zweiten Verbindungselements (512) an die Buchse (200; 450) gekoppelt ist,
wobei vorzugsweise das zweite Verbindungselement (512) zumindest zwei Bohrungen (530b, 532b, 534b) zum entsprechenden Aufnehmen zumindest eines Teils einer Buchse (200; 450) dahindurch und zumindest eines Teils einer Bohrerführung (502, 504) dahindurch aufweist.

22. Bohrerführungsanordnung nach Anspruch 21, wobei die zumindest zwei Bohrungen (530a, 532a, 534a) des ersten Verbindungselements (510) durch eine erste Distanz (D₁) getrennt sind, und die zumindest zwei Bohrungen (530b, 532b, 534b) des zweiten Verbindungselements (512) durch eine zweite Distanz (D₂) getrennt sind, wobei die erste Distanz (D₁) größer ist als die zweite Distanz (D₂), und wobei das zweite Verbindungselement (512) näher am distalen Ende des Vorwärtsendes (222) der Buchse (200; 450) ist als das erste Verbindungselement (510).

23. Bohrerführungsanordnung nach Anspruch 21, wobei die zumindest zwei Bohrungen (530a, 532a, 534a) des ersten Verbindungselements (510) durch eine erste Distanz (D₁) getrennt sind, und die zumindest zwei Bohrungen (530b, 532b, 534b) des zweiten Verbindungselements (512) durch eine zweite Distanz (D₂) getrennt sind, wobei die erste Distanz (D₁) größer ist als die zweite Distanz (D₂), und wobei das erste Verbindungselement (510) näher am distalen Ende des Vorwärtsendes (222) der Buchse (200; 450) ist als das zweite Verbindungselement (512).

24. Bohrerführungsanordnung nach Anspruch 20, wobei das erste Verbindungselement (510) des Weiteren eine Finnen-Bohrung (536) umfasst, welche eingerichtet ist, zumindest einen Teil einer Finne (514) dahindurch aufzunehmen, wobei zumindest ein Teil der Finne (514), eingerichtet ist, mit zumindest einem Teil eines Lochs (352) oder einer Ausnehmung (354) zusammenzuwirken, wenn die Buchse (200; 450) mit einer Knochenplatte (350) zusammenwirkt.

25. Bohrerführungsanordnung nach einem der Ansprüche 18 bis 24, des Weiteren umfassend eine zweite Bohrerführung (504), welche an die Buchse (200; 450) gekoppelt ist, wobei die zweite Bohrerführung (504) eingerichtet ist, einen Bohrer (400) aufzunehmen und zu führen,
wobei vorzugsweise die zweite Bohrerführung (504) an die erste Bohrerführung (502) gekoppelt ist, und
wobei weiter vorzugsweise die erste und zweite Bohrertührung (502, 504) mittels eines ersten Verbindungselements (510) an die Buchse (200; 450) gekoppelt sind, und
wobei noch weiter vorzugsweise das erste Verbindungselement (510) zumindest drei Bohrungen (530a, 532a, 534a) aufweist zum entsprechenden Aufnehmen zumindest eines Teils der ersten Bohrerführung (502) dahindurch, zumindest eines Teils der zweiten Bohrerführung (504) dahindurch und zumindest eines Teils der Buchse (200; 450) dahindurch.

26. Bohrerführungsanordnung nach Anspruch 25, wobei das erste Verbindungselement (510) des Weiteren eine Finnen-Bohrung (536) umfasst, welche eingerichtet ist, zumindest einen Teil einer Finne (514) dahindurch aufzunehmen, und wobei zumindest ein Teil der Finne (514) eingerichtet ist, mit zumindest einem Teil eines Lochs (352) oder einer Ausnehmung (354) zusammenzuwirken, wenn die Buchse (200; 450) mit einer Knochenplatte (350) zusammenwirkt, oder
wobei die erste Bohrerführung (502) und die zweite Bohrerführung (504) des Weiteren mittels eines zweiten Verbindungselements (512) an die Buchse (200; 450) gekoppelt sind.

27. Bohrerführungsanordnung nach Anspruch 26, wobei das zweite Verbindungselement (512) zumindest drei Bohrungen (530b, 532b, 534b) aufweist zum entsprechenden Aufnehmen zumindest eines Teils der ersten Bohrerführung (502) dahindurch, zumindest eines Teils der zweiten Bohrerführung (504) dahindurch und zumindest eines Teils der Buchse (200; 450) dahindurch.

28. Bohrerführungsanordnung nach Anspruch 27, wobei die Bohrungen (530a, 532a, 534a) des ersten Verbindungselements (510), welche erste und zweite Bohrerführungen (502, 504) aufnehmen, durch eine erste Distanz (D₁) getrennt sind, und die Bohrungen (530b, 532b, 534b) des zweiten Verbindungselements (512), welche erste und zweite Bohrerführungen (502, 504) aufnehmen, durch eine zweite Distanz (D₂) getrennt sind, wobei die erste Distanz (D₁) größer ist als die zweite Distanz (D₂), und wobei das zweite Verbindungselement (512) näher am distalen Ende des Vorwärtsendes (222) der Buchse (200; 450) ist als das erste Verbindungselement (510).

29. Bohrerführungsanordnung nach Anspruch 27, wobei die Bohrungen (530a, 532a, 534a) des ersten Verbindungselements (510), welche erste und zweite Bohrerführungen (502, 504) aufnehmen, durch eine erste Distanz (D₁) getrennt sind, und die Bohrungen (530b, 532b, 534b) des zweiten Verbindungselements (512), welche erste und zweite Bohrerführungen (502, 504) aufnehmen, durch eine zweite Distanz (D₂) getrennt sind, wobei die erste Distanz (D₁) größer ist als die zweite Distanz (D₂), und wobei das erste Verbindungselement (510) näher am distalen Ende des Vorwärtsendes (222) der Buchse (200; 450) ist als das zweite Verbindungselement (512).

30. Bohrerführungsanordnung nach einem der Ansprüche 18 bis 29, wobei die erste Bohrerführung (502) eine Längsachse aufweist, und wobei, wenn die Buchse (200; 450) an eine Knochenplatte (350) verriegelt ist, die Längsachse der ersten Bohrerführung (502) im Allgemeinen zu einem ersten Knochenbefestigungsloch (352) der Knochenplatte (350) ausgerichtet ist.

31. Bohrerführungsanordnung nach Anspruch 30, des Weiteren umfassend eine zweite Bohrerführung (504), welche eingerichtet ist, einen Bohrer (400) aufzunehmen und zu führen, und an die Buchse (200; 450) gekoppelt ist, wobei die zweite Bohrerführung (504) eine Längsachse aufweist, und wobei, wenn die Buchse (200; 450) an eine Knochenplatte (350) verriegelt ist, die Längsachse der zweiten Bohrerführung (504) im Allgemeinen zu einem zweiten Knochenbefestigungsloch (352) der Knochenplatte (350) ausgerichtet ist.

32. Bohrerführungsanordnung nach einem der Ansprüche 18 bis 31, wobei die Ausnehmung (354) der Knochenplatte (350) zumindest einen geformten Bereich (356) und einen Schlitz (360) enthält.

33. Teilesatz zur Verwendung beim Bohren von Knochen, umfassend:
(a) eine Bohrerführungsanordnung (5; 500) nach einem der Ansprüche 1 bis 32;
(b) zumindest eine erste und zweite Bohrerführung (502, 504), welche an die Buchse (200; 450) gekoppelt werden können; und
(c) zumindest ein erstes und zweites Verbindungselement (510, 512) zum Koppeln zumindest einer Bohrerführung (502, 504) an die Buchse (200; 450).

34. Teilesatz nach Anspruch 33, wobei zumindest die erste und zweite Bohrerführung (502, 504) verschiedene Längen aufweisen, oder
wobei zumindest die erste und zweite Bohrerführung (502, 504) verschiedene Durchmesser aufweisen.

35. Teilesatz nach Anspruch 33 oder 34, wobei zumindest das erste und zweite Verbindungselement (510, 512) jeweils eine Bohrung (532, 534) zum Aufnehmen zumindest einer Bohrerführung (502, 504) dahindurch und eine Bohrung (530) zum Aufnehmen einer Buchse (200; 450) dahindurch aufweisen, wobei die Bohrungen (530a, 532a, 534a) des ersten Verbindungselements (510) eine erste Anordnung aufweisen und die Bohrungen (530b, 532b, 534b) des zweiten Verbindungselements (512) eine zweite Anordnung aufweisen, und wobei die erste Anordnung im Wesentlichen verschieden von der zweiten Anordnung ist.

## Revendications

1. Ensemble de guide-foret (5 ; 500) comprenant :
un corps cylindrique d'alignement (150) ayant une extrémité proximale (174) et une extrémité distale (172) ;
une douille (200 ; 450) configurée pour recevoir de manière coulissante le corps cylindrique d'alignement (150), la douille (200 ; 450) ayant une extrémité avant radialement expansible (222) et une extrémité proximale, l'extrémité avant (222) étant configurée pour pouvoir être insérée à l'intérieur d'un trou (352) ou évidement (354) dans une lame osseuse (350) ;
un bouton de libération (100) ; et
un mécanisme d'engrenage mobile (50 ; 550) ayant une première patte (52), une seconde patte (54) et une queue (56),
la première patte (52) du mécanisme d'engrenage (50 ; 550) étant raccordée au corps cylindrique d'alignement (150),
la deuxième patte (54) du mécanisme d'engrenage (50 ; 550) étant adaptée pour maintenir le corps cylindrique d'alignement (150) en position,
la queue (56) du mécanisme d'engrenage (50 ; 550) pouvant être actionnée par un utilisateur pour déplacer sélectivement le mécanisme d'engrenage (50 ; 550),
dans lequel le mouvement du mécanisme d'engrenage (50 ; 550) fait coulisser le corps cylindrique d'alignement (150) par rapport à la douille (200 ; 450) pour étendre radialement l'extrémité avant (222) afin de bloquer de manière libérable la douille (200 ; 450) sur la lame (350),
**caractérisé en ce que** le mécanisme d'engrenage mobile (50 ; 550) est un mécanisme d'encliquetage mobile, et **en ce que** le bouton de libération (100 ; 600) a des dentelures (102), et la seconde patte (54) du mécanisme d'encliquetage (50 ; 550) a des cliquets (58) configurés et adaptés pour mettre en prise les dentelures (102) afin de maintenir le corps cylindrique d'alignement (150) en position.

2. Ensemble de guide-foret selon la revendication 1, dans lequel le mécanisme d'encliquetage (50 ; 550) est monté de manière pivotante, ou bien
dans lequel le bouton de libération (100) est monté de manière pivotante, ou bien
dans lequel le mécanisme d'encliquetage (50 ; 550) pivote de manière incrémentielle, ou bien
dans lequel le corps cylindrique d'alignement (150) comprend un alésage de passage (185) de l'extrémité distale (172) à l'extrémité proximale (174).

3. Ensemble de guide-foret selon la revendication 1 ou 2, dans lequel le corps cylindrique de foret d'alignement (150) comprend en outre une première section cylindrique creuse (156), une deuxième section cylindrique creuse (158) et une troisième section cylindrique creuse (160), dans lequel le diamètre annulaire (X₂₄) de la troisième section cylindrique (160) est au moins égal au diamètre annulaire (X₁₈) de la deuxième section cylindrique (158), et dans lequel le diamètre annulaire (X₁₈) de la seconde section cylindrique (158) est au moins égal au diamètre annulaire (X₁₂) de la première section cylindrique (156), ou bien
dans lequel le diamètre annulaire (X₂₄) de la troisième section cylindrique (160) est constant le long de la ligne médiane (180) de la section cylindrique (160).

4. Ensemble de guide-foret selon l'une quelconque des revendications 1 à 3, dans lequel le corps cylindrique d'alignement (150) comprend en outre deux crêtes (152, 154) au niveau de l'extrémité proximale (174),
dans lequel de préférence, la surface externe du corps cylindrique de foret d'alignement (150) a un épaulement (164) au niveau de l'extrémité distale (172), ou bien
dans lequel de préférence le diamètre externe (X₂₆) de la troisième section cylindrique (160) se rétrécit progressivement pour former une forme conique.

5. Ensemble de guide-foret selon l'une quelconque des revendications 1 à 4, dans lequel la douille (200 ; 450) comprend en outre des doigts (210 ; 570) radialement expansibles.

6. Ensemble de guide-foret selon la revendication 5, dans lequel les doigts (210 ; 570) forment un col circonférentiel (208) radialement expansible et adoptent une disposition non expansée vers l'intérieur dans un état détendu,
dans lequel, de préférence, la douille (200 ; 450) comprend en outre un épaulement (212) adjacent au col circonférentiel (208) radialement expansible.

7. Ensemble de guide-foret selon la revendication 5 ou 6, dans lequel l'extrémité distale (222) de la douille (200 ; 450) comprend une extrémité progressivement rétrécie avec le diamètre interne et le diamètre externe de l'extrémité rétrécie qui diminue dans la direction de la pointe, ou bien
dans lequel la douille (200 ; 450) est réalisée avec une seule pièce de matériau de construction unitaire, ou bien
dans lequel la douille (200 ; 450) comprend en outre une broche pour fixer l'ensemble de guide-foret (5 ; 500) sur une lame osseuse (350), ou bien
dans lequel la douille (200 ; 450) comprend au moins une fente verticale au-dessus de la partie circulaire (206) sur la douille (200 ; 450).

8. Ensemble de guide-foret selon l'une quelconque des revendications 1 à 7, dans lequel le corps cylindrique d'alignement (150) est configuré pour recevoir et guider un foret (400), ou bien
comprenant en outre au moins un premier guide-foret (502) couplé à la douille (200 ; 450), dans lequel au moins le premier guide-foret (502) est configuré pour recevoir et guider un foret (400).

9. Ensemble de guide-foret (5 ; 500) selon l'une quelconque des revendications 1 à 8, comprenant en outre :
un ensemble de poignée (250 ; 650) pour la préhension par un utilisateur ; et
un support de bras double (10 ; 610) ayant des première et seconde parties (14, 16), la première partie (14) du support de bras double (10 ; 610) étant raccordée de manière fixe à l'extrémité proximale (222) de la douille (200 ; 450) et la seconde partie (16) du support de bras double (10 ; 610) étant raccordée de manière fixe à l'élément de poignée (250 ; 650) ;
dans lequel le bouton de libération (100) est raccordé de manière mobile à l'élément de poignée (250 ; 650) ; et
la première patte (52) du mécanisme d'encliquetage (50 ; 550) est raccordée de manière pivotante au support de bras double (10 ; 610) et en outre raccordée au corps cylindrique d'alignement (150).

10. Ensemble de guide-foret selon la revendication 9, dans lequel le support de bras double (10 ; 610) est en forme de L, ou bien
dans lequel le support de bras double (10 ; 610) est fixé à la douille (200 ; 450) par un procédé de fixation choisi dans le groupe comprenant le soudage, l'ajustage par friction et l'ajustage à la presse, ou bien
dans lequel la première partie (14) du support de bras double (10 ; 610) et la direction axiale de l'ensemble d'alignement (15 ; 515) forment un angle de l'ordre d'environ 75 degrés à environ 120 degrés, ou bien
dans lequel la seconde partie (16) du support de bras double (10 ; 610) et la première partie (14) du support de bras double (10 ; 610) forment un angle de l'ordre d'environ 90 degrés à environ 150 degrés, ou bien
dans lequel le support de bras double (10 ; 610) est une construction monolithique solidaire, ou bien
dans lequel la deuxième partie (16) du support de bras double (10 ; 610) et l'élément de poignée (250 ; 650) forment un angle de l'ordre d'environ 90 degrés à environ 150 degrés, ou bien
dans lequel l'axe longitudinal (280) de l'élément de poignée (250 ; 650) se trouve dans le même plan que l'axe longitudinal (24) de la première partie (14) du support de bras double (10 ; 610), et l'axe longitudinal (26) de la seconde partie (16) du support de bras double (10 ; 610), ou bien
dans lequel l'axe longitudinal (280) de l'élément de poignée (250 ; 650) est parallèle à l'axe longitudinal (24) de la première partie (14) du support de bras double (10 ; 610), ou bien
dans lequel le support de bras double (10 ; 610) est raccordé de manière immobile à la douille (200 ; 450) au niveau de l'extrémité proximale (242) de la douille (200 ; 450) et le support de bras double (10 ; 610) est raccordé de manière immobile à l'élément de poignée (250 ; 650), ou bien
dans lequel le support de bras double (10 ; 610) a un espace ouvert et le mécanisme d'encliquetage (50 ; 550) est disposé dans l'espace ouvert et monté de manière pivotante à l'intérieur de ce dernier.

11. Ensemble de guide-foret selon la revendication 9 ou 10, dans lequel l'élément de poignée (250 ; 650) a une extrémité avant (254) et une extrémité arrière, et dans lequel le support de bras double (10 ; 610) et l'élément de poignée (250 ; 650) sont raccordés de manière fixe au niveau de l'extrémité avant (254) de l'élément de poignée (250 ; 650).

12. Ensemble de guide-foret selon l'une quelconque des revendications 9 à 11, dans lequel le mécanisme d'encliquetage (50 ; 550) est en forme de Y,
dans lequel de préférence la première patte (52) du mécanisme d'encliquetage (50 ; 550) s'étend en outre au-delà du point de pivot formant une pince étau en forme de C (60) ; dans lequel la pince étau en forme de C (60) saisit le corps cylindrique d'alignement (150) entre les deux crêtes (152, 154) sur le corps cylindrique d'alignement (150), et
dans lequel, en outre, de préférence le plan de la pince étau en forme de C (60) fait un angle aigu avec l'axe longitudinal (64) de la première patte (52) du mécanisme d'encliquetage en forme de Y (50 ; 550), ou bien
dans lequel, en outre, de préférence l'angle aigu est de l'ordre d'environ 25 degrés à 45 degrés.

13. Ensemble de guide-foret selon l'une quelconque des revendications 9 à 12, dans lequel l'élément de poignée (250 ; 650) est fixé sur le support de bras double (10 ; 610) avec une broche (20) qui est perpendiculaire à l'axe (280) de l'élément de poignée (250 ; 650), ou bien
dans lequel ladite extrémité avant expansible (222) de ladite douille (200 ; 450) est de forme circulaire, et pouvant être librement insérée et retirée dudit trou (352) ou évidement (354) dans une position contractée, et mettant en prise la lame osseuse (350) lorsqu'elle est dans une position expansée.

14. Ensemble de guide-foret selon l'une quelconque des revendications 9 à 13, dans lequel ladite extrémité avant (222) radialement expansible comprend une pluralité de parties de doigt (210 ; 570).

15. Ensemble de guide-foret selon la revendication 14, dans lequel :
ladite extrémité avant (222) radialement expansible de la douille (200 ; 450) comprend un épaulement (212), un col (208), et un bord (214) en saillie vers l'extérieur, disposé vers l'avant dudit col (208) ;
dans lequel ledit col (208) et le bord (214) recouvrent ensemble une longueur qui est légèrement plus longue que l'épaisseur de la paroi de trou (352) ou d'évidement (354), et le bord (214) vient en butée contre la surface du côté de l'os de ladite lame (350).

16. Ensemble de guide-foret selon la revendication 15, dans lequel l'élément de poignée (250 ; 650) comprend une première cavité (256) et une seconde cavité (258) au niveau de l'extrémité avant (254), dans lequel la première cavité (526) loge un ressort de compression (272), et la seconde cavité (258) loge la seconde partie (16) du support de bras double (10 ; 610).

17. Ensemble de guide-foret selon l'une quelconque des revendications 9 à 16, dans lequel le corps cylindrique d'alignement (150) est configuré pour recevoir et guider un foret (400), ou bien
comprenant en outre au moins un premier guide-foret (502) couplé à la douille (200 ; 450), dans lequel au moins le premier guide-foret (502) est configuré pour recevoir et guider un foret (400).

18. Ensemble de guide-foret selon l'une quelconque des revendications 1 à 17, comprenant en outre :
un premier guide-foret (502) couplé à la douille (200 ; 450), dans lequel le premier guide-foret (502) est configuré pour recevoir et guider un foret (400).

19. Ensemble de guide-foret selon la revendication 18, dans lequel le premier guide-foret (502) est couplé à la douille (200 ; 450) par un premier élément de raccordement (510).

20. Ensemble de guide-foret selon la revendication 19, dans lequel le premier élément de raccordement (510) a au moins deux alésages (530a, 532a, 534a) pour recevoir respectivement au moins une partie d'une douille (200 ; 450) à travers ce dernier et au moins une partie d'un guide-foret (502, 504) à travers ce dernier.

21. Ensemble de guide-foret selon la revendication 20, dans lequel le premier guide-foret (502) est en outre couplé à la douille (200 ; 450) par un deuxième élément de raccordement (512),
dans lequel de préférence, le second élément de raccordement (512) a au moins deux alésages (530b, 532b, 534b) pour recevoir respectivement au moins une partie d'une douille (200 ; 450) à travers ce dernier et au moins une partie d'un guide-foret (502, 504) à travers ce dernier.

22. Ensemble de guide-foret selon la revendication 21, dans lequel les au moins deux alésages (530a, 532a, 534a) du premier élément de raccordement (510) sont séparés par une première distance (D₁), et les au moins deux alésages (530b, 532b, 534b) du deuxième élément de raccordement (512) sont séparés par une seconde distance (D₂), dans lequel la première distance (D₁) est supérieure à la seconde distance (D₂), et dans lequel le deuxième élément de raccordement (512) est plus près de l'extrémité distale de l'extrémité avant (222) de la douille (200 ; 450) que le premier élément de raccordement (510).

23. Ensemble de guide-foret selon la revendication 21, dans lequel les au moins deux alésages (530a, 532a, 534a) du premier élément de raccordement (510) sont séparés par une première distance (D₁), et les au moins deux alésages (530b, 532b, 534b) du second élément de raccordement (512) sont séparés par une seconde distance (D₂), dans lequel la première distance (D₁) est supérieure à la seconde distance (D₂), et dans lequel le premier élément de raccordement (510) est plus près de l'extrémité distale de l'extrémité avant (222) de la douille (200 ; 450) que le second élément de raccordement (512).

24. Ensemble de guide-foret selon la revendication 20, dans lequel le premier élément de raccordement (510) comprend en outre un alésage d'ailette (536) configuré pour recevoir au moins une partie d'ailette (514) à travers ce dernier, dans lequel au moins une partie de l'ailette (514) est configurée pour mettre en prise au moins une partie d'un trou (352) ou évidement (354) lorsque la douille (200 ; 450) met en prise une lame osseuse (350).

25. Ensemble de guide-foret selon l'une quelconque des revendications 18 à 24, comprenant en outre un second guide-foret (504) couplé à la douille (200 ; 450), dans lequel le second guide-foret (504) est configuré pour recevoir et guider un foret (400),
dans lequel de préférence, le second guide-foret (504) est couplé au premier guide-foret (502), et
dans lequel, en outre, de préférence, les premier et second guide-forets (502, 504) sont couplés à la douille (200 ; 450) par un premier élément de raccordement (510), et
dans lequel, encore en outre, de préférence, le premier élément de raccordement (510) a au moins trois alésages (530a, 532a, 534a) pour recevoir respectivement au moins une partie du premier guide-foret (502) à travers ce dernier, au moins une partie du second guide-foret (504) à travers ce dernier, et au moins une partie de la douille (200 ; 450) à travers ce dernier.

26. Ensemble de guide-foret selon la revendication 25, dans lequel le premier élément de raccordement (510) comprend en outre un alésage d'ailette (536) configuré pour recevoir au moins une partie de l'ailette (514) à travers ce dernier, et dans lequel au moins une partie de l'ailette (514) est configurée pour mettre en prise au moins une partie d'un trou (352) ou évidement (354) lorsque la douille (200 ; 450) met en prise une lame osseuse (350), ou bien
dans lequel le premier guide-foret (502) et le second guide-foret (504) sont en outre couplés à la douille (200 ; 450) par un second élément de raccordement (512).

27. Ensemble de guide-foret selon la revendication 26, dans lequel le second élément de raccordement (512) a au moins trois alésages (530b, 532b, 534b) pour recevoir respectivement au moins une partie du premier guide-foret (502) à travers ce dernier, au moins une partie du second guide-foret (504) à travers ce dernier, et au moins une partie de la douille (200 ; 450) à travers ce dernier.

28. Ensemble de guide-foret selon la revendication 27, dans lequel les alésages (530a, 532a, 534a) du premier élément de raccordement (510) recevant les premier et second guide-forets (502, 504) sont séparés par une première distance (D₁), et les alésages (530b, 532b, 534b) du second élément de raccordement (512) recevant des premier et second guide-forets (502, 504) sont séparés par une seconde distance (D₂), dans lequel la première distance (D₁) est supérieure à la seconde distance (D₂), et dans lequel le second élément de raccordement (512) est plus proche de l'extrémité distale de l'extrémité avant (222) de la douille (200 ; 450) que le premier élément de raccordement (510).

29. Ensemble de guide-foret selon la revendication 27, dans lequel les alésages (530a, 532a, 534a) du premier élément de raccordement (510) recevant les premier et second guide-forets (502, 504) sont espacés par une première distance (D₁) et les alésages (530b, 532b, 534b) du second élément de raccordement (512) recevant les premier et second guide-forets (502, 504) sont séparés par une seconde distance (D₂), dans lequel la première distance (D₁) est supérieure à la seconde distance (D₂), et dans lequel le premier élément de raccordement (510) est plus proche de l'extrémité distale de l'extrémité avant (222) de la douille (200 ; 450) que le second élément de raccordement (512).

30. Ensemble de guide-foret selon l'une quelconque des revendications 18 à 29, le premier guide-foret (502) ayant un axe longitudinal, et dans lequel lorsque la douille (200 ; 450) est bloquée sur une lame osseuse (350), l'axe longitudinal du premier guide-foret (502) est généralement aligné avec un premier trou de fixation d'os (352) de la lame osseuse (350).

31. Ensemble de guide-foret selon la revendication 30, comprenant en outre un second guide-foret (504) configuré pour recevoir et guider un foret (400) et couplé à la douille (200 ; 450), le second guide-foret (504) ayant un axe longitudinal, et dans lequel lorsque la douille (200 ; 450) est bloquée sur une lame osseuse (350), l'axe longitudinal du second guide-foret (504) est généralement aligné avec un second trou de fixation d'os (352) de la lame osseuse (350).

32. Ensemble de guide-foret selon l'une quelconque des revendications 18 à 31, dans lequel l'évidement (354) de la lame osseuse (350) comprend au moins une surface profilée (356) et une fente (360).

33. Kit destiné à être utilisé pour percer des os, comprenant :
(a) un ensemble de guide-foret (5 ; 500) selon l'une quelconque des revendications 1 à 32 ;
(b) au moins des premier et second guide-forets (502, 504) pouvant être couplés à la douille (200 ; 450) ; et
(c) au moins des premier et second éléments de raccordement (510, 512) pour coupler au moins un guide-foret (502, 504) à la douille (200 ; 450).

34. Kit selon la revendication 33, dans lequel au moins les premier et second guide-forets (502, 504) ont des longueurs différentes, ou bien
dans lequel au moins les premier et second guide-forets (502, 504) ont des diamètres différents.

35. Kit selon la revendication 33 ou 34, dans lequel au moins les premier et second éléments de raccordement (510, 512) ont chacun un alésage (532, 534) pour recevoir au moins un guide-foret (502, 504) à travers ces derniers, et un alésage (530) pour recevoir une douille (200 ; 450) à travers ces derniers, dans lequel les alésages (530a, 532a, 534a) du premier élément de raccordement (510) ont un premier agencement, et les alésages (530b, 532b, 534b) du second élément de raccordement (512) ont un second agencement, et dans lequel le premier agencement est sensiblement différent du second agencement.
